# EUROPEAN PATENT APPLICATION

(11) **EP 2 772 760 A1**
(43) Date of publication of application: **03.09.2014**
(21) Application number: 12842861.2
(22) Date of filing: 24.10.2012
(51) Int. Cl.: G01N 33/579, C12Q 1/37, G01N 21/49, G01N 21/59, G01N 21/82

(54) **METHOD FOR MEASURING PHYSIOLOGICALLY ACTIVE SUBSTANCE OF BIOLOGICAL ORIGIN, AND MICROPARTICLES AND EXTRACT FOR USE IN SAID METHOD**

(30) Priority: 26.10.2011 JP 2011234680; 26.10.2011 JP 2011234681
(71) Applicant: Kowa Company Ltd., Naka-ku Nagoya-shi Aichi 460-8625 (JP)
(72) Inventor: YABUSAKI, Katsumi, Tokyo 103-8433 (JP)
(74) Representative: Chettle, Adrian John
(86) International application number: PCT/JP2012/077509
(87) International publication number: WO 2013/062013

(57) **Abstract**

Provided is a technique which can detect a physiologically active substance of biological origin or measure the concentration of the physiologically active substance of biological origin with higher accuracy or within a shorter period even when a sample that contains the physiologically active substance of biological origin at an extremely low concentration or a sample that contains a component capable of interfering with an AL is used. Microparticles capable of adsorbing a specific physiologically active substance, e.g., an endotoxin, is dispersed in a sample containing the specific physiologically active substance to cause the adsorption of the specific physiologically active substance onto the microparticles, thereby concentrating the specific physiologically active substance in the sample. Subsequently, the microparticles are separated and collected, then are reacted with an AL to cause an aggregation reaction, and the aggregation is detected by an optical means. In this manner, the measurement of the specific physiologically active substance can be carried out.

## Description

### Technical Field

The present invention relates to a measurement method for detecting a physiologically active substance of biological origin having a property of being gelated by reaction with AL such as endotoxin and β glucan in a sample containing the physiologically active substance, or measuring the concentration thereof, and microparticles used in the method. In addition, the present invention relates to a method for measuring a physiologically active substance of biological origin in a medical device by extracting the physiologically active substance of biological origin adhering to the medical device, adsorbing the physiologically active substance of biological origin onto the microparticles, and then mixing the microparticles with a Limulus reagent (AL reagent) which is a reagent for measuring the physiologically active substance, and detecting aggregation of AL under a stirring condition with an optical technique whereby to measure the physiologically active substance, and an extraction liquid used in the method.

### Background Art

Endotoxin is a lipopolysaccharide present in a cell wall of a Gram-negative bacterium and is the most typical pyrogen. If a transfusion, a medicine for injection, the blood or the like contaminated with endotoxin is introduced into the human body, endotoxin may induce severe side effects such as fever and shock. Therefore, it has been required that the above-mentioned medicine or the like is kept not to be contaminated with endotoxin. On the other hand, measurement of endotoxin in the blood of a patient having sepsis may contribute to prevention or treatment of severe endotoxin shock.

In addition, β glucan is a polysaccharide that constructs the cell membrane that is characteristic to a fungus. Measurement of β glucan is effective for screening of infectious diseases due to a variety of fungi including not only fungi which are frequently observed in general clinical practices such as Candida, Aspergillus, and Cryptococcus, but also rare fungi, and the like.

By the way, serine protease activated by endotoxin, β glucan or the like exists in a hemocyte extract of limulus (hereinafter, also referred to as "AL: Amoebocyte lysate".). Then, when AL reacts with endotoxin or β glucan, a coagulogen existing in AL is hydrolyzed to a coagulin by an enzyme cascade by the serine protease activated according to the amount of endotoxin or β glucan, and the coagulin is associated to form an insoluble gel. With the use of this property of AL, it is possible to detect endotoxin or β glucan with high sensitivity. In recent years, with use of this, methods of using AL in detection or concentration measurement of endotoxin and the like were invented.

As a method for performing detection or concentration-measurement of this physiologically active substance of biological origin (hereinafter, also referred to as "the predetermined physiologically active substance".) detectable by AL such as endotoxin and β glucan (hereinafter, also simply referred to as the "measurement of the predetermined physiologically active substance".), the followings are known. For example, the method includes a turbidimetric method including analyzing a sample by measuring the turbidity of the sample by a reaction between AL and the predetermined physiologically active substance; a colorimetric method decomposing a synthetic substrate that is added by an enzyme in AL activated by the predetermined physiologically active substance to develop a color; and the like. However, the turbidity of AL itself or the coloring by a synthetic substrate when a low concentration of the predetermined physiologically active substance is applied, is extremely weak, and thus the lower limit for measuring the physiologically active substance in these measurement methods was 0.001 EU/mL (EU: endotoxin unit) or so.

On the other hand, the measurement sensitivity for some of dialysis fluids is insufficient with a conventional turbidimetric method or a colorimetric method. For example, according to Standard 2008 of the Japanese Society for Dialysis Therapy, the endotoxin concentration of ultrapure dialysis fluid or dialysis fluid for substitution is less than 0.001 EU/mL. Thus, establishment of a measurement method for achieving further higher sensitivity is urgently needed. As the measurement method for achieving the higher sensitivity, reported are a method developed from the turbidimetric method in which the sample is stirred (stirring turbidimetric method), a method in which gel microparticles formed in a stirred sample are detected with light scattering method (light scattering method), a method in which AL is bound onto the microparticles whereby to reinforce the aggregation reaction (AL-bound bead method), and the like. Then, a technique for performing measurement of the predetermined physiologically active substance in 0.0001 EU/mL or less within a practical time, was also reported in recent years.

By the way, AL is gelated by the chain reaction of multiple proteases (serine protease). Accordingly, in the case where a substance inhibiting or promoting serine protease of AL (interference substance) in a sample specimen is contained, exact quantification of the predetermined physiologically active substance may not be able to be performed unless the sample is sufficiently diluted or the interference substance is removed. In contrast, a batch method has been suggested in which a lipid A-binding peptide selected from an amino acid, a polypeptide and a random peptide library, which adsorbs a predetermined physiologically active substance, or the like is bound to a bead-shaped carrier, and mixed with a sample containing the predetermined physiologically active substance whereby to immobilize the predetermined physiologically active substance onto a carrier. In this batch method, the carrier immobilized with the predetermined physiologically active substance, is reacted with AL, and the turbidity change of AL by gelation or the action of the predetermined physiologically active substance is measured.

Examples of the batch method include PyroSep method in which PyroSep capable of binding endotoxin is bound to cellulose or agarose bead to form a column, and a sample containing a large quantity of endotoxin is passed through this (see Non-Patent Literature 1). In this PyroSep method, the column after the sample passage is washed with alkali-side buffer to dissociate endotoxin, and then is reacted with AL, and endotoxin is quantified with kinetic turbidimetric method. This makes it possible to detect 0.5 EU/mL of endotoxin in 60 minutes or so.

Other examples of the batch method may include the random peptide library method (Patent Literature 1). In this method, a peptide from a random peptide library recognizing lipid A, which is the activity core of the endotoxin (for example, XYSSS (X = K, R, or H)) is bound to Propionyl chloride functionalized silica gel (Sigma, Aldrich) having 200 to 400 mesh (50 to 100 µm). In addition, in this method, the silica gel after the binding is mixed with a sample containing endotoxin to adsorb the endotoxin onto the bead. Then, the silica gel is washed with centrifuge 6 times, and then the endotoxin is measured with Endospecy.

However, any binding carrier used in the method was large, and had low light scattering property and was made of a fragile material (agarose, cellulose, silica gel). On the other hand, with the measurement method enabling short time measurement such as the AL-bound bead method, it requires stirring of the sample, and a binding carrier having high refractive index and high strength is demanded since the predetermined physiologically active substance is optically measured. Accordingly, it was difficult to apply the method to the AL-bound bead method and the like, and it was difficult to drastically shorten the measurement time of the predetermined physiologically active substance with the method.

In addition, if endotoxin enters the human body, it may induce severe side effects such as fever and shock as described above. Therefore, a medicine for injection or a medical device such as an injection-related device must not contain endotoxin in a concentration higher than a certain concentration, or in an amount higher than a certain quantity. Thus, endotoxin contaminating the medicine for injection or the medical device is strictly managed. Herein, the medical device represents a device installed in the body of a patient in the treatment, an injection device such as a syringe and an injection needle, a filter tube or bag in contact with a medicine for injection or transfusion, and the like.

Herein, it is known that endotoxin adhering to a medical device is difficult to extract with water (Patent Literature 5). In contrast, it is regarded that a method of extracting endotoxin from a medical device with an amine-containing compound or an extraction liquid suitably containing albumin and the like, and measuring the endotoxin concentration in the extraction liquid after the extraction, is effective. However, extraction of endotoxin adhering to the device depends on the size of the device, and requires use of a large quantity of the extraction liquid, and thus the endotoxin is diluted by the extraction liquid. In that case, the method had problems that it is difficult to measure the endotoxin since the endotoxin is thin, and the detection time is long.

### Citation List

### Patent Literature

Patent Literature 1: WO 2007/060769 A
Patent Literature 2: Japanese Patent Application Laid-Open (JP-A) No. 2009-118854A
Patent Literature 3: JP-A No.2009-150723
Patent Literature 4: WO2008/038329 A
Patent Literature 5: JP-A No.5-255405

### NON-PATENT LITERATURE

Non-Patent Literature 1: Masakazu Tsuchiya, "Topic 20 PyroSep method, Talking of LAL", Technical report of Wako Pure Chemical Industries, Ltd., Wako Pure Chemical Industries, Ltd., p. 21, No. 3, Vol. 63, July 15, 1995
Non-Patent Literature 2: Kenichi Tanamoto, "Endotoxin and management of quality of medicine", Report of National Institute of Health Sciences, National Institute of Health Sciences, pp. 19 to 33, No. 126,2008
Non-Patent Literature 3: Written by James R. Robinson, et al. (other 4 persons), "Depyrogenation by Microporous Membrane Filters", US, PDA technical report, p. 54, July 1985

### Summary of Invention

### Technical Problem

The present invention has been developed in view of the above-mentioned problems. An object of the present invention is to provide a technology for achieving more accurate and short-time detection and concentration measurement of a predetermined physiologically active substance of biological origin even with respect to a sample having extremely low concentration of a physiologically active substance of biological origin or a sample containing an ingredient having interfering action against AL. In addition, an object of the present invention is to provide a measurement method for achieving more accurate and short-time measurement of a physiologically active substance of biological origin adhering to a medical device, and an extraction liquid used in the measurement method.

### Solution to Problem

The greatest feature of the present invention, which is intended to solve the above-mentioned problems, is to perform measurement of a predetermined physiologically active substance by dispersing microparticles having an affinity to the predetermined physiologically active substance in a sample containing the predetermined physiologically active substance, to adsorb the predetermined physiologically active substance onto the microparticles, and then collecting the microparticles and reacting the microparticles with an AL whereby to induce an aggregation reaction, and detecting the aggregation with an optical technique.

More specifically, the present invention provides a method for measuring a physiologically active substance of biological origin by producing a mixed solution of an AL reagent containing AL which is a hemocyte extract of limulus and a sample containing a predetermined physiologically active substance of biological origin, and detecting protein aggregation or gelation induced by a reaction between AL and the physiologically active substance in the mixed solution with an optical technique while stirring the mixed solution whereby to detect the physiologically active substance in the sample or measure the concentration of the physiologically active substance, which is characterized by
dispersing microparticles capable of adsorbing the physiologically active substance on the surface in the sample whereby to adsorb the physiologically active substance on the surface of the microparticle, and then isolating the microparticles from the sample,
producing a mixed solution of the AL reagent and the microparticle adsorbed with the physiologically active substance on the surface, and
detecting aggregation or gelation of the microparticle in the mixed solution of the AL reagent and the microparticle with an optical technique.

In the present invention, microparticles containing a material having low optical transparency or high refractive index and sufficient strength against mechanical external force are mixed with a sample containing a predetermined physiologically active substance, and the predetermined physiologically active substance are adsorbed onto the microparticle. The sample at the time may have a property that the concentration of the predetermined physiologically active substance is extremely low and/or a property of containing an interference substance affecting the reaction of AL. Then, the microparticles adsorbed with the predetermined physiologically active substance, are isolated and collected by spontaneous sedimentation or an operation such as centrifugal isolation or filtration by a filter, and as necessary, the microparticles are washed with a liquid not containing the predetermined physiologically active substance. Then, the microparticle and AL are reacted whereby to induce the aggregation reaction, and optical properties such as the light transmittance and the intensity or peak number of light-scattering light are acquired, whereby to perform measurement of the predetermined physiologically active substance.

In the present invention, measurement of the predetermined physiologically active substance may be performed by adsorbing the predetermined physiologically active substance adhering to the medical device onto microparticles having an affinity to the predetermined physiologically active substance, and then isolating and collecting the microparticles and reacting the microparticles with AL whereby to induce the aggregation reaction, and detecting the aggregation with an optical technique.

More specifically, the present invention provides a method for measuring a predetermined physiologically active substance in a medical device by producing a mixed solution of an AL reagent containing AL which is a hemocyte extract of limulus and an extraction liquid containing the predetermined physiologically active substance extracted from the medical device, and detecting protein aggregation or gelation induced by a reaction between AL and the predetermined physiologically active substance in the mixed solution with an optical technique while stirring the mixed solution, whereby to detect the predetermined physiologically active substance in the extraction liquid or measure the concentration of the predetermined physiologically active substance, wherein the method includes
dispersing the microparticles capable of adsorbing the predetermined physiologically active substance on the surface in the extraction liquid whereby to adsorb the predetermined physiologically active substance onto the surface of the microparticle, and then isolating the microparticles from the extraction liquid,
producing a mixed solution of the AL reagent and the microparticles adsorbed with the predetermined physiologically active substance on the surface, and
detecting aggregation or gelation of the microparticles in the mixed solution of the AL reagent and the microparticles with an optical technique.

In other words, the predetermined physiologically active substance adhered to the medical device is extracted with an extraction liquid in which the microparticles having an affinity to the predetermined physiologically active substance are dispersed, and adsorbed onto the microparticles. The microparticle is specifically a microparticle adsorbing the predetermined physiologically active substance, which contains a material having low optical transparency or high refractive index and sufficient strength against mechanical external force as a main ingredient, or one of the ingredients. Then, the microparticles adsorbed with the predetermined physiologically active substance are isolated and collected by spontaneous sedimentation or an operation such as centrifugal isolation or filtration by a filter, and as necessary, the microparticles are washed with a liquid not containing the predetermined physiologically active substance. Then, the microparticle and AL are reacted whereby to induce the aggregation reaction, and optical properties such as light transmittance and the intensity or the peak number of light-scattering light are acquired, whereby to perform measurement of the predetermined physiologically active substance adhered to the medical device.

The microparticle in the present invention may be composed of one or multiple materials selected from PyroSep, polymixin B, polylysine, polyornithine, polyarginine, polyhistidine, aminosilane, chitosan, an anti-endotoxin antibody, an anti-endotoxin aptamer, a material in a random peptide library, a metal oxide such as alumina, titania, silica, zirconia and hydroxyapatite, and a natural or synthetic mineral such as kaolin, montmorillonite, manganese oxide and mica.

In other words, the adsorption material for adsorbing the predetermined physiologically active substance may be either an organic compound having excellent adsorption ability for the predetermined physiologically active substance such as endotoxin and having high selectivity, or an inorganic substance not having high selectivity but having excellent adsorption ability for the predetermined physiologically active substance such as endotoxin. Examples of the organic compound include PyroSep, polymixin B, polylysine, polyornithine, polyarginine, polyhistidine, aminosilane, chitosan, an anti-endotoxin antibody, an anti-endotoxin aptamer, an endotoxin adsorption peptide selected from a random peptide library (for example, XYSSS (X = K, R, or H) exemplified in Patent Literature 2 (JP-A No. 2009-118854)), and the like. In addition, examples of the inorganic substance include a metal oxide such as alumina, titania, silica, zirconia and hydroxyapatite, and a natural or synthetic mineral such as kaolin, montmorillonite, manganese oxide and mica.

The organic compound may be granulated as the material itself if possible, or may be bound onto the microparticles of an organic or inorganic binding carrier by electrostatic adsorption or chemical binding and made to particles. In other words, the microparticle in the present invention may be formed by binding an adsorption material selectively adsorbing the physiologically active substance onto the surface of the carrier microparticle.

The adsorption material in this case may be one or multiple materials selected from PyroSep, polymixin B, polylysine, polyornithine, polyarginine, polyhistidine, aminosilane, chitosan, an anti-endotoxin antibody, an anti-endotoxin aptamer and a material in a random peptide library.

In addition, examples of the organic carrier microparticle in this case include microparticles of polystyrene latex, polyethylene, nylon, cellulose, agarose, polyvinyl alcohol, an acrylic resin, and the like. In addition, examples of the inorganic carrier microparticle include microparticles of simple substances such as alumina, titania, silica, zirconia and hydroxyapatite, or composite-material microparticles thereof (for example, silica alumina, alumina titania and the like), and microparticles of a natural or synthetic mineral such as kaolin, montmorillonite, manganese oxide and mica, and the like.

At least one kind or more kinds of the microparticles may be selected and used, or 2 or more kinds of different materials may be mixed and used, regardless of being an organic compound or an inorganic compound, or being a microparticle of the material itself or a microparticle in which the adsorption material is bound on the carrier microparticle. Furthermore, the following two points should be noted in selecting the material. First of all, the point is that a particle having low light transmittance (including colored particles) or a particle having high light transmittance but having high refractive index is effective in detecting the microparticle. Next, the point is that the microparticle needs to have a sufficient strength since if the particles are fragile, destruction of the particles under stirring is induced by the external force from the stirring.

The microparticle carrier is desirably a ceramic microparticle that has high refractivity and is hard such as alumina and titania in comprehensive consideration of the optical properties, the mechanical strength of the particle and the like demanded in the present invention. Since the ceramic microparticle is easily sterilized with hot-air, it is possible to prevent contamination of endotoxin, and since the ceramic microparticle has moderate specific gravity, the collection of the microparticle by centrifugal isolation is easy.

These microparticles can be desirably isolated and collected with spontaneous sedimentation or a simple operation. It is difficult to collect microparticles having a very small particle diameter. Conversely, if the microparticles have a large particle diameter, (1) a dispersion property of the microparticles in water or an extraction liquid is low. (2) Effective surface area for adsorbing the microparticles is low. (3) It is difficult for the predetermined physiologically active substance to invade into the inside of the particle. For such reason, the adsorption ability for the predetermined physiologically active substance decreases. (4) In the case where the microparticle after having adsorbed the predetermined physiologically active substance should be washed, water in the inside of the microparticle is hardly discharged, and thus the washing effect decreases. Furthermore, in reacting the microparticle having adsorbed the predetermined physiologically active substance with AL to induce the co-aggregation, smaller particle diameter as possible leads to shortening of the measurement time. From these, the particle diameter of the microparticles is desirably 5 nm to 50 µm, and further desirably 10 nm to 20 µm.

As a technique for concentrating the microparticles having adsorbed the predetermined physiologically active substance, considered may be spontaneous sedimentation, precipitation by centrifugal isolation, and in addition, filtration by a filter or hollow fibers, and the like. In fact, the particles may be concentrated by selecting at least one kind from these, or by combining them. A technique of precipitating the microparticles by centrifugal isolation is desirable in consideration of shortening of the concentration time, the simplicity and the improvement of the concentration rate.

In addition, a surfactant may be added to the microparticles in the present invention. In addition, the microparticle may be formed so as to be covered by the adsorption material on the whole surface.

Herein, in manufacturing the microparticles having adsorbed the predetermined physiologically active substance in the present invention, or in adsorbing the predetermined physiologically active substance of biological origin adhered to the medical device onto the microparticles, the case is considered where a material capable of adsorbing the predetermined physiologically active substance is bound to the surface of the carrier microparticle. In this case, it may be considered that the material capable of adsorbing the predetermined physiologically active substance cross-links multiple carrier particles, and the microparticles aggregate locally. In contrast, a means for preventing aggregation of the microparticles or a means for dispersing the aggregated microparticles may be added. The former may be exemplified by addition of a substance having surfactant action, binding of a material capable of adsorbing the predetermined physiologically active substance to the carrier microparticles in an excessive amount to cover the whole surface of the carrier microparticle, and the like. In addition, the latter may be exemplified by grant of mechanical impact or vibration, or grinding with a mill or homogenizer whereby to disperse the aggregated microparticles.

In addition, the microparticle in the present invention may contain a material having an affinity to coagulogen in the AL reagent. Alternatively, the microparticle may be formed to contain a material having an affinity to coagulogen, and may be previously bound with coagulogen.

By the way, with the AL-bound bead method, AL, particularly, coagulogen in AL is bound onto the microparticles and the coagulogen on the microparticle changes to coagulin and rapid aggregation is caused whereby to enable fast aggregation reaction. As a result, a method enabling short time measurement is realized. Also in the microparticle used in the present invention, coagulogen may be previously immobilized onto the microparticle as necessary. Alternatively, a substance having an affinity to AL or coagulogen may be immobilized in advance to a portion of the microparticle. Furthermore, the microparticle itself may have affinity to both of endotoxin and coagulogen. For example, alumina or titania has high affinity to endotoxin and at the same time, has high affinity to a protein. Thus, microparticles of them can be used for the object of the present invention.

In addition, some of organic materials adsorbing the predetermined physiologically active substance such as endotoxin have high affinity to a protein as well. For example, polylysine, polyornithine, polyarginine and the like adsorb a protein electrostatically. By being bound with these organic materials, the microparticle has an affinity to both of the predetermined physiologically active substance and coagulogen. By using this microparticle, it is possible to concentrate endotoxin and make the microparticles participate in the aggregation reaction whereby to shorten the measurement time.

In addition, in the case where an interference substance affecting the reaction between the AL and the physiologically active substance is contained in the sample or the extraction liquid in the present invention, the interference substance may be removed by washing the microparticles after being isolated from the sample or the extraction liquid.

By the way, AL is gelated by the chain reaction of multiple proteases (serine protease). Accordingly, in the case where a substance inhibiting or promoting serine protease of AL (interference substance) is contained in the sample or in the extraction liquid, exact measurement of the predetermined physiologically active substance may be not performed unless the sample or the extraction liquid is sufficiently diluted or the interference substance is removed as described above. In contrast, in the case where such interference substance is contained in the sample or the extraction liquid in the present invention, the microparticles after being isolated from the sample or the extraction liquid are washed, whereby to remove the interference substance.

According to this, it is possible to remove the interference substance in the sample or in the extraction liquid at a step before mixing the microparticles having adsorbed and concentrated the predetermined physiologically active substance with the AL reagent. By this, it is possible to remove the influence of the interference substance, and it is possible to perform more accurate measurement of the predetermined physiologically active substance.

In addition, in the present invention, beads adsorbed with the predetermined protein contained in the hemocyte extract of limulus on the surface, may be dispersed in advance in the AL reagent.

Herein, in the AL-bound bead method described above, an AL reagent is prepared in which the protein contained in AL is bound to or adsorbed on the bead, and this AL reagent is applied with a sample containing the predetermined physiologically active substance. This makes it possible to early produce large aggregation mass by the associated beads, and promote aggregation or gelation in the mixed solution of the AL reagent and the sample or the extraction liquid. Accordingly, by making the AL reagent being mixed with the microparticles adsorbed with the predetermined physiologically active substance on the surface according to the present invention, and dispersed with the beads according to the AL-bound bead method, it is possible to exert synergistic effects of the present invention and the AL-bound bead method, and further perform short-time measurement of the predetermined physiologically active substance.

In addition, in the present invention, the physiologically active substance of biological origin may be endotoxin or β glucan.

Then, it is possible to perform more accurate detection or concentration-measurement of endotoxin that is the most representative pyrogenic substance, and suppress entry of a transfusion, a medicine for injection, the blood and the like contaminated with endotoxin into the human body to induce side effects. Similarly, it is possible to perform more accurate detection or concentration-measurement of β glucan, and perform more accurate screening of infectious diseases due to a variety of fungi including not only fungi which are frequently observed in general clinical practices such as Candida, Aspergillus, and Cryptococcus, but also rare fungi.

In addition, the present invention may be microparticles capable of adsorbing the physiologically active substance on the surface, wherein the microparticles used in any method for measuring a physiologically active substance of biological origin described above.

In addition, the present invention provides an extraction liquid that is used in the method for measuring a physiologically active substance of biological origin in a medical device described above, and extracts the predetermined physiologically active substance from the medical device, which is characterized that the microparticles capable of adsorbing the predetermined physiologically active substance on the surface are previously dispersed in the extraction liquid.

Furthermore, the means for resolving the problems of the present invention described above can be used in combination as possible.

### Advantageous Effects of Invention

According to the present invention, it is possible to measure a physiologically active substance of biological origin in a sample in the state of being concentrated and adsorbed on the microparticles, and it is possible to measure the physiologically active substance of biological origin at a lower concentration than a conventional method. In addition, by using aggregation between the microparticles, it is possible to induce fast aggregation reaction, and measure a low concentration of the physiologically active substance of biological origin in a short time. In addition, even in the case where a substance interfering with the AL reaction exists, it is possible to remove the substance by suitably washing the microparticles isolated and collected from the sample, and it is possible to perform measurement of the physiologically active substance of biological origin in a higher accuracy.

In addition, according to the present invention, it is possible to extract a predetermined physiologically active substance adhered to a medical device, and concentrate and adsorb the predetermined physiologically active substance on a microparticle participating in the aggregation reaction by itself, and as a result, it is possible to measure the predetermined physiologically active substance adhered to the medical device at a up to lower concentration than a conventional method. In addition, particularly in extracting a predetermined physiologically active substance from a medical device, a large quantity of an extraction liquid is necessary. However, in the present invention, it is possible to concentrate the physiologically active substance of biological origin adsorbed onto the microparticles by centrifuge and the like together with the microparticles, and thus it is possible to avoid a disadvantage that the physiologically active substance of biological origin is diluted by the amount of the extraction liquid and cannot be measured. In addition, even in the case where a substance interfering with the AL reaction exists in a medical device, it is possible to remove the substance by suitably washing the microparticles isolated and collected from the sample, and it is possible to perform measurement of a physiologically active substance of biological origin adhered to the medical device in higher accuracy.

### Brief Description of Drawings

Fig. 1 is a view illustrating comparison of endotoxin removal (adsorption) actions by various inorganic materials.
Fig. 2 is a graph illustrating the relationship between the concentration of endotoxin adsorbed onto various inorganic materials and the detection time.
Fig. 3 is a graph illustrating the relationship between the volume of a sample containing endotoxin and the detection time in the case where endotoxin-adsorption microparticles are used.
Figs. 4(a) and 4(b) are graphs illustrating the adsorption reaction temperature and the relationship between the adsorption reaction time and the detection time when a sample containing endotoxin is reacted with the endotoxin-adsorption microparticles.
Fig. 5 is a graph illustrating the relationship between the endotoxin concentration and the detection time in the case where coagulogen is previously bound to the endotoxin-adsorption microparticle and the measurement is performed.
Figs. 6(a) and 6(b) are graphs illustrating the change of the reaction speed at the time of endotoxin measurement in the case where the endotoxin-adsorption microparticles and the AL-bound beads are concomitantly used.
Fig. 7 is a graph illustrating the detecting time at the time of endotoxin measurement in the case where the endotoxin-adsorption microparticles and the AL-bound beads are concomitantly used.
Fig. 8 is a graph illustrating the relationship between the concentration of β glucan and the detection time in the case where measurement of β glucan is performed using β glucan adsorption microparticles.
Fig. 9 is a graph illustrating the relationship between the endotoxin concentration of the sample and the detection time in the case where endotoxin-adsorptive microparticles were used and in the case where endotoxin-adsorptive microparticles were not used.
Figs. 10(a) and 10(b) are views illustrating the flow of the endotoxin measurement in the case where endotoxin-adsorptive microparticles were used and in the case where endotoxin-adsorptive microparticles were not used.
Fig. 11 is a graph illustrating the temporal change of the light transmittance in the case where endotoxin-adsorptive microparticles were used and in the case where endotoxin-adsorptive microparticles were not used.

### Description of Embodiments

Hereinafter, embodiments of the present invention are specifically illustrated using the figures. Furthermore, in the description below, endotoxin or β glucan is taken as examples and explained as the predetermined physiologically active substance, but the description below can be also applied to other physiologically active substances of biological origin.

As the microparticle in the present embodiment, considered may be (1) inorganic microparticles having low selectivity for endotoxin, but being capable of adsorbing endotoxin and coagulogen in AL, and (2) inorganic carrier microparticles bound with an organic adsorption material having high selectivity and affinity for endotoxin. In the former case, the selectivity for endotoxin is low, and thus the function as an endotoxin-adsorption material decreases in the case where the ingredients contained in the sample are more strongly bound to endotoxin than the inorganic microparticles, or in the case where the other ingredients than endotoxin in the sample bind to inorganic microparticles. On the other hand, the latter has high binding specificity to endotoxin, and thus can sufficiently exert the ability as the adsorption material.

Next, preparation of microparticles adsorbing endotoxin will be explained. As the carrier microparticle, ceramic microparticles such as alumina and titania, which are inorganic materials having affinity to both of endotoxin and coagulogen in AL, may be used. Alternatively, an organic adsorption material capable of adsorbing endotoxin selectively in high affinity may be bound onto the surface of these ceramic microparticles. In addition, these ceramic microparticles may be previously treated with hot air whereby to completely remove endotoxin.

In the case where an organic endotoxin-adsorption material is bound to microparticles of alumina or titania, polymixin B, polylysine, polyarginine, polyhistidine, polyornithine, anti-endotoxin/lipid A antibody, anti-endotoxin/lipid A nucleic acid aptamer, a peptide for endotoxin/lipid A selected from a random peptide library, and the like may be used. Since alumina or titania has high affinity to a protein or nucleic acid as described above, it is possible to bind the adsorption material without chemical binding.

However, it is assumed that in some of the adsorption materials, the binding is dissociated only with electrostatic binding to alumina or titania, or that the binding ability to the predetermined physiologically active substance such as endotoxin and glucan decreases by the binding. In such a case, a functional group capable of chemically binding to an organic compound may be introduced onto the surface of alumina or titania, through which the adsorption material is chemically bound. As the functional group introduced in this case, for example, silane coupling agents may be mentioned.

Specifically, the coupling agent may be, for example, an aminosilane coupling agent having an amino group and capable of chemically binding to a carboxyl group contained in the adsorption material. In addition, the coupling agent may be an epoxysilane coupling agent having an epoxy group and capable of chemically binding to an amino group contained in the adsorption material. Furthermore, the coupling agent may be an isocyanate silane coupling agent or an isothiocyanate having an isocyanate group or an isothiocyanate group and capable of chemically binding to an amino group in the adsorbing agent. Furthermore, the chemical substance used herein may be a substance capable of chemically binding to a portion of the adsorbing agent and capable of binding to the inorganic compound such as titania and alumina, and is not limited to the silane coupling agents exemplified herein.

In addition, when these endotoxin-adsorption materials are bound to the microparticles, an AL ingredient may be added in a small amount in advance as necessary, or either one of the endotoxin-adsorption material and the AL ingredient may be bound previously, and then the other may be applied and bound. Alternatively, microparticles bound with the endotoxin-adsorption material and microparticles bound with AL or coagulogen may be prepared individually, and mixed in an appropriate mixing ratio and used.

In the case where the affinity of the organic adsorption material to the inorganic carrier microparticle is strong when the organic adsorption material is bound to the inorganic carrier microparticle, the adsorption material may become a crosslinking agent (cross-linking reaction) or the charge on the microparticle may be neutralized, thus resulting in aggregation of a portion or all of the microparticles. Thus, the organic adsorption material may be reacted in an excessive amount with respect to the inorganic carrier microparticle. In addition, it is also effective to add a substance having surfactant action in the preparation process.

This substance having surfactant action desirably has no interfering action or little interfering action to the AL reaction. For example, non-ionic surfactants represented by Triton-based, Tween-based, Nonidet-based, or Pluronic-based surfactants and the like, or albumin frequently used as a stabilizer for AL or an extracting agent for endotoxin at the time of the endotoxin measurement, or the like is desirably used. However, the substance having surfactant action is not intended to be limited to the specific examples.

In addition, in the case where the particles aggregate despite such means, strong impact may be given after precipitation of the particles to scatter the precipitate, or a mill or homogenizer may be used to make it microparticles. For example, the endotoxin-adsorptive microparticles prepared by mixing and binding excessive polylysine with alumina microparticles, and then performing removal with operations of washing and centrifugal isolation as in Preparation Example 3, have excellent light refractivity, and also have small particle diameter, and thus can be used in the endotoxin measurement method pertaining to the present invention.

In determining the endotoxin concentration using the microparticles of the present invention, a dispersion liquid containing the prepared microparticles is mixed with a sample, and the adsorption reaction is performed for an appropriate time, and then the microparticles are precipitated by a method such as centrifugal isolation. In the case where an ingredient of the sample has an interfering action with respect to the AL reaction, the microparticles are washed with appropriate water (injection water and the like) not containing endotoxin at this time. In the washing, the microparticles in the mixed solution of water and the microparticles are precipitated by a method such as centrifugal isolation, and the supernatant is removed. Then, the remaining microparticle is re-suspended with water, and precipitated again by centrifugal isolation, and the supernatant is removed. By suitably repeating this operation, the microparticles can be washed.

In addition, by adding an ingredient having a surfactant effect to a dispersion liquid of the microparticles mixed with the sample, it is possible to stabilize the microparticles at the time of the endotoxin-adsorption reaction. In addition, since endotoxin is dispersed by the surfactant action, it is possible to effectively adsorb endotoxin onto the microparticles. Similarly, also when the microparticles adsorbed with endotoxin is mixed with AL, an ingredient having a surfactant action is preferably added. If the microparticle itself or the endotoxin-adsorption material bound to the microparticles rapidly binds to coagulogen in AL, an aggregation reaction having nothing to do with the aggregation reaction by endotoxin may occur.

For specific procedures of the measurement, 100 µL dispersion liquid of the microparticle manufactured in Preparation Example 3 is mixed with 1 mL of the sample and reacted at room temperature as described in Example 1. Then, the microparticles are isolated and collected from the mixed solution by centrifugal isolation and dispersed with 50 µL of the dispersion liquid. The dispersed microparticle and 50 µL of the AL reagent are put into the glass vessel manufactured in Preparation Example 1 and reacted, and the aggregation reaction at the time is optically measured whereby to perform the endotoxin measurement. The aggregation reaction in the mixed solution of the microparticle and the AL reagent can be measured with a device having a stirring system for measuring light transmittance, for example, a device for the stirring turbidimetric method EX-100 (manufactured by Kowa Company, Ltd.) or a device for analysis of time-resolved particle size distribution using laser light scattering EX-300 (manufactured by Kowa Company, Ltd.), or the like. In addition, a micro plate reader having a stirring function of the sample and for achieving measurement of light transmittance or scattering light may be used.

### <Preparation Example 1> (Endotoxin-free glass vessel)

In the light transmittance measurement method, a special vessel made of glass having 6 mm ϕ and 50 mm length and provided with a stirring bar made of stainless steel for stirring the sample (0.75 mm ϕ, 3.5 mm length) was used. On the other hand, in the laser light scattering particle counting method, a special vessel made of glass having 7 mm ϕ and 50 mm length and provided with a stirring bar made of stainless steel for stirring the sample (1 mm ϕ, 5 mm length) was used. The openings of these glass vessels were covered with an aluminum foil. Further, those packaged with an aluminum foil in subdivision to 20 pieces were put into a hot-air treatment can made of iron, and heat-treated for 3 hours at 250°C, whereby to thermally decompose endotoxin.

### <Preparation Example 2> (Inorganic microparticle having endotoxin-adsorption ability)

Silica microparticles SFP-20M manufactured by DENKI KAGAKU KOGYO KABUSHIKI KAISHA (primary particle diameter: 20 nm), alumina microparticles ASFP-20 manufactured by DENKI KAGAKU KOGYO KABUSHIKI KAISHA (primary particle diameter: 20 nm), alumina microparticles AA03 manufactured by Sumitomo Chemical Co., Ltd (primary particle diameter: 400 nm), and titania microparticles AEROXIDE P25 manufactured by Nippon Aerosil Co., Ltd. (primary particle diameter: 20 nm) were put into glass tubes in subdivision and the openings of the glass tubes were covered with an aluminum foil. Further, several pieces were collected and put into a glass beaker, and the openings were further covered with an aluminum foil, and hot-air treated for 30 minutes at 250°C whereby to completely inactivate endotoxin which is a risk of causing contamination. This was dispersed in a dispersion liquid to the predetermined concentration whereby to prepare microparticles having no endotoxin selectivity but having adsorption ability.

### <Preparation Example 3> (Alumina microparticle bound with organic material having endotoxin-adsorption ability)

The alumina microparticles ASFP-20 manufactured by DENKI KAGAKU KOGYO KABUSHIKI KAISHA prepared in Preparation Example 2 were dispersed in an aqueous solution of 0.1% poly-L-lysine (Poly-L-K (hereinafter, it may be briefly written as PLK)) (manufactured by Sigma, Aldrich) in 10 mg/mL of the concentration of the microparticle. The dispersion liquid was slowly stirred at room temperature for 2 hours whereby to adsorb poly-L-lysine onto the alumina microparticle. Then, the dispersion liquid was centrifuged and the supernatant was removed, and the residue was washed three times with distilled water for injection not containing endotoxin, and finally dispersed in an aqueous solution of a non-ionic surfactant Pluronic F68 (30%) in 0.25 mg/mL of the converted concentration of the beads of the material whereby to prepare microparticles having selective endotoxin-adsorption ability (PLK-ASFP).

### <Preparation Example 4> (Silica microparticle bound with organic material having endotoxin-adsorption ability)

The silica microparticles SFP-20M manufactured by DENKI KAGAKU KOGYO KABUSHIKI KAISHA prepared in Preparation Example 2 were dispersed in an aqueous solution of 0.1% poly-L-lysine (manufactured by Sigma, Aldrich) in 10 mg/mL of the concentration of the microparticle. The dispersion liquid was slowly stirred at room temperature for 2 hours whereby to adsorb poly-L-lysine onto the silica microparticle. Then, the dispersion liquid was centrifuged and the supernatant was removed, and the residue was washed three times with distilled water for injection not containing endotoxin, and finally dispersed in distilled water for injection in 1.0 mg/mL of the converted concentration of the microparticle of the material whereby to prepare microparticles having endotoxin-adsorption ability (PLK-SFP).

### <Preparation Example 5> (Alumina microparticle bound with organic material having endotoxin-adsorption ability and coagulogen)

50 µL of an AL reagent (ES-II Multi reagent manufactured by Wako Pure Chemical Industries, Ltd.) was added to 0.5 mL of the alumina microparticles bound with poly-L-lysine prepared by the method of Preparation Example 3 (PLK-ASFP, concentration: 10 mg/mL), and the mixture was heat-treated at 60°C for 10 minutes. Then, the mixture was washed once with distilled water for injection not containing endotoxin to prepare microparticles bound with the endotoxin-adsorption material and AL (PLK=LAL-ASFP).

### <Preparation Example 6> (Titania microparticle bound with organic material having endotoxin-adsorption ability)

The titania microparticles AEROXIDE P25 manufactured by Nippon Aerosil Co., Ltd. prepared in Preparation Example 2 were dispersed in an aqueous solution of 0.1% poly-L-lysine (manufactured by Sigma, Aldrich) in 10 mg/mL of the concentration. The dispersion liquid was slowly stirred at room temperature for 2 hours whereby to bind poly-L-lysine to the titania microparticles. Then, the dispersion liquid was centrifuged and the supernatant was removed, and the residue was washed three times with distilled water for injection not containing endotoxin, and finally dispersed in a non-ionic surfactant Triton X-100 (0.2%) in 0.25 mg/mL of the converted concentration of the microparticle of the material whereby to prepare microparticles having selective endotoxin-adsorption ability (PLK-P25).

### <Preparation Example 7> (Alumina microparticles bound with coagulogen)

The alumina microparticles ASFP-20M manufactured by DENKI KAGAKU KOGYO prepared in Preparation Example 2 were dispersed in distilled water for injection in 30 mg/mL of the concentration. 500 µL of a dispersion liquid of P25 was added to 500 µL of an AL reagent (ES-II Multi reagent manufactured by Wako Pure Chemical Industries, Ltd.) and well mixed, and heat-treated at 80°C for 20 minutes. Then, the mixture was washed once with 1 mL of water for injection and dispersed in water for injection whereby to give alumina microparticles bound with AL (AL-ASFP).

Furthermore, poly-L-lysine was used as the endotoxin-adsorption material in Preparation Examples. As polylysine, there are α poly-D/L-lysine, an original polypeptide in which an α-position amino group and a carboxyl group are linked by an amide bond (= peptide bond), and ε poly-L-lysine in which an ε-position amino group and a carboxyl group are linked by an amide bond (not a peptide bond in the narrow sense). Since the L form is easily decomposed by enzymes contained in a cell, the D form, which is hardly decomposed, may be used as necessary. Although the present embodiment using poly-L-lysine will be explained, either poly-L-lysine or poly-D-lysine may be used as the adsorption material.

Next, endotoxin measurements with respect to a sample containing thin endotoxin and a sample having endotoxin interfering action by the microparticles having the endotoxin-adsorption ability will be explained.

### <Example 1> (Endotoxin-concentration action by inorganic microparticle and quantification by aggregation reaction)

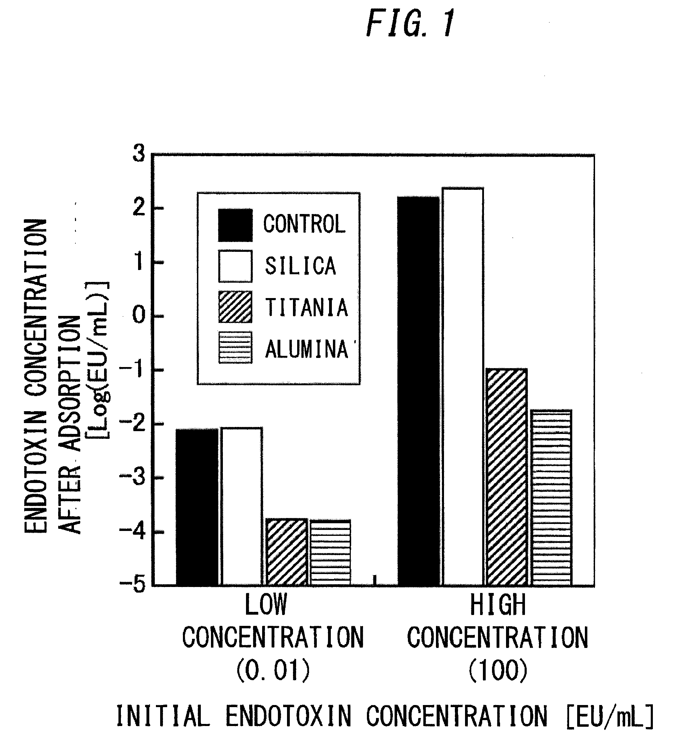

The endotoxin-adsorption action was evaluated using the microparticles prepared in Preparation Example 2. 1 mg of any one of SFP-20M (silica), AA03 (alumina) and AEROXIDE P25 (titania) was dispersed in 1 mL of an aqueous solution of high-concentration endotoxin (100 EU/mL), or a low-concentration endotoxin (0.01 EU/mL) to give 1 mg/mL of the concentration of the microparticles. This was stirred for 30 minutes at room temperature, and then the microparticles were precipitated by centrifugal isolation (15,000 x g, 5 minutes), and 50 µL of the supernatant and 50 µL of AL were mixed whereby to induce the aggregation reaction, which was measured with the stirring turbidimetric method (Kowa EX-100). The results are shown in Fig. 1. As shown in Fig. 1, alumina and titania adsorbed endotoxin, and adsorbed and removed endotoxin until the concentration became one hundredth or less of the initial concentration in any concentration of the high and the low concentrations. However, silica did not adsorb endotoxin, and did not change from the reference results (initial concentration).

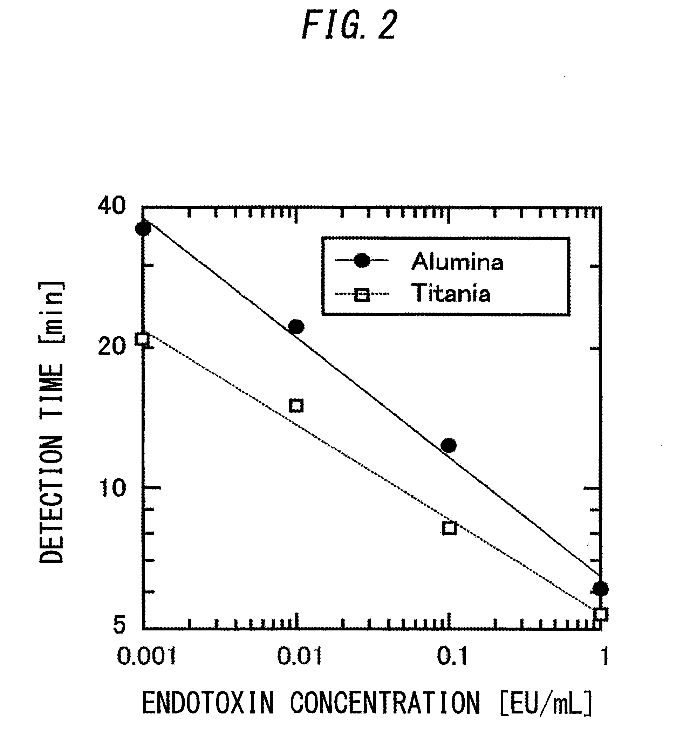

Next, with respect to alumina and titania having endotoxin-adsorption ability, the microparticles of them were added to dilution series of endotoxin (1 mL) (containing 1 mg/mL of AA03, 0.25 mg/mL of P25 and 0.2% of Triton X-100 as a surfactant), and the adsorption reaction was performed for 20 minutes at room temperature. The beads were isolated and collected by centrifugal isolation and then re-suspended in an aqueous solution of Triton X-100 (0.02%, 50 µL), and this was mixed and reacted with AL (manufactured by Wako Pure Chemical Industries, Ltd., ES-II Multi reagent, 50 µL). Measurement of the aggregation was performed with the stirring turbidimetric method (EX-100). As shown in Fig. 2, in the microparticles of any one of the materials, the adsorbed endotoxin and AL were reacted to induce the aggregation reaction, and the detection time was shorter as the concentration of the applied endotoxin was higher.

As described above, a few of the inorganic microparticles can adsorb various organic substances effectively, and also bind to endotoxin and coagulogen in AL. Thus, it is considered that first, the microparticles are firstly applied with endotoxin to concentrate the endotoxin on the microparticles, and then applied with AL, whereby to quickly bind AL (coagulogen) onto the surface of the microparticles not adsorbed with endotoxin, and then endotoxin on the microparticle binds to C factor in AL, whereby to increase the activity of AL, and change coagulogen to coagulin, which induces the aggregation reaction involving the microparticles.

### <Example 2> (Investigation of adsorption condition)

In order to examine whether or not the endotoxin-adsorption microparticles of the present invention can concentrate endotoxin from a sample containing endotoxin, and participate (involved) in aggregation when mixed with an AL reagent, the endotoxin-adsorption microparticles were used and reacted with various volumes of aqueous solutions of endotoxin, and the endotoxin-concentration action was evaluated. With respect to the various volumes of aqueous solutions of endotoxin, the alumina microparticles (ASFP-20, 20 µL of 1 mg/mL) described in Preparation Example 1, and the silica microparticles bound with poly-L-lysine (adsorbed and immobilized) as the endotoxin-adsorption material (PLK-SFP, 20 µL of 10 mg/mL) described in Preparation Example 4 were mixed with 0.01 EU/mL of the endotoxin-containing solution, and reacted at room temperature for 20 minutes.

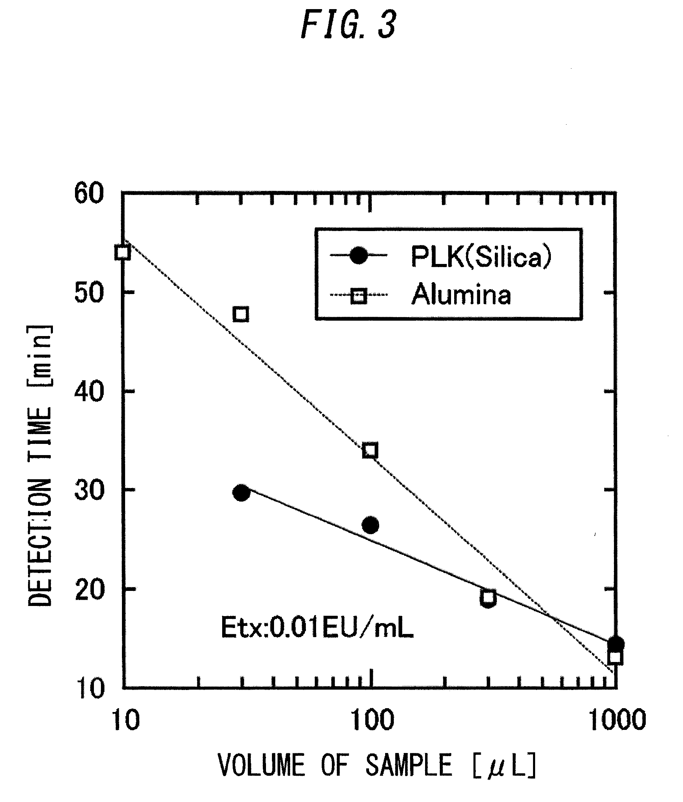

The microparticles were precipitated by centrifugal isolation, and then re-suspended in an aqueous solution of a non-ionic surfactant Triton X-100 (0.02%, 50 µL), and this was mixed and reacted with an AL reagent (manufactured by Wako Pure Chemical Industries, Ltd., ES-II Multi reagent, 50 µL). The results are shown in Fig. 3. From the figure, it is understood that the endotoxin detection time is shorter as the liquid volume of the aqueous solution of endotoxin applied is greater in any one of the endotoxin-adsorption microparticles. This showed that endotoxin can be concentrated even from a large quantity of the liquid volume by these microparticles.

In addition, it was shown that coagulogen in AL binds to the microparticles at a very early stage of the AL aggregation, and endotoxin adsorbed (captured) on the microparticle activates AL, and as a result thereof, the microparticles are cross-linked through the produced coagulins, whereby these microparticles can induce steep aggregation reaction. This aggregation reaction is similar to that described in Patent Literature (JP-A No. 2009-150723), and the aggregation reaction mainly involving the microparticles occurs earlier than the aggregation reaction of AL itself by endotoxin, which makes it possible to drastically shorten the measurement time, and measure lower concentration of endotoxin.

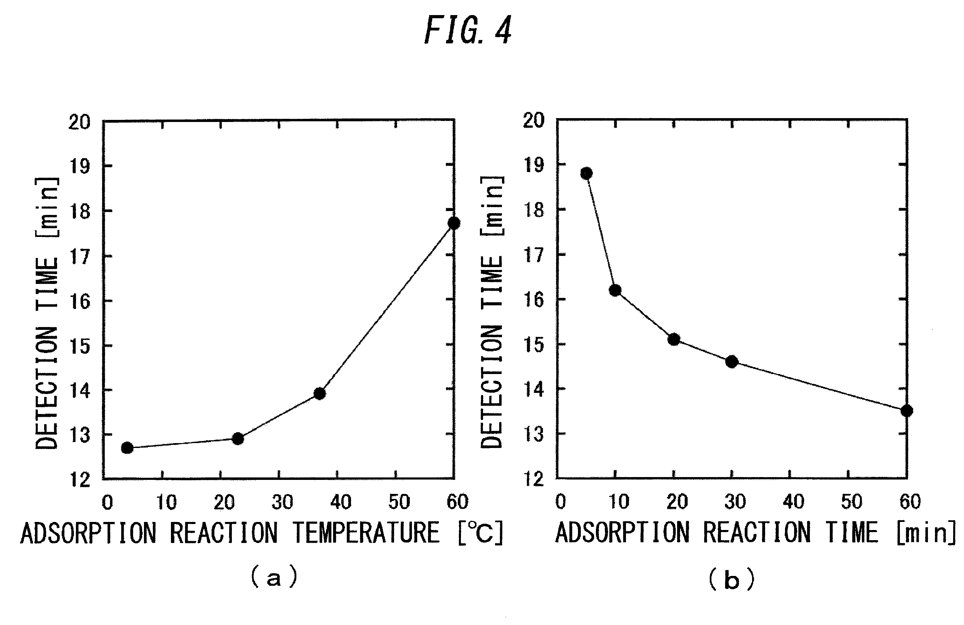

Next, in order to investigate optimal reaction temperature in the adsorption reaction, the endotoxin-adsorption reaction was performed at various temperatures, and then the microparticle having adsorbed endotoxin were isolated and collected, and dispersed in the dispersion liquid (50 µL). Then, and this was reacted with an AL reagent (manufactured by Wako Pure Chemical Industries, Ltd., ES-II Multi reagent, 50 µL) and the aggregation reaction was measured with EX-100, and the endotoxin detection times were compared. The used microparticles were the alumina microparticles bound with poly-L-lysine (PLK-ASFP) described in Preparation Example 3. The applied endotoxin was 1 mL in 0.01 EU/mL of the concentration. The reaction time was 30 minutes. The results are shown in Fig. 4(a). As shown in the figure, the detection time was shorter as the temperature was lower. From this, it is understood that the adsorption reaction between endotoxin and the microparticles is a physical adsorption reaction by an adsorption material having high selectivity for endotoxin (since the reaction is faster as the temperature is lower, and the adsorption reaction usually generates adsorption heat).

Further, the minimum reaction time necessary for the adsorption reaction was examined. Experiments the same as the investigation of the reaction temperature condition were performed applying various reaction times with the reaction temperature immobilized at room temperature (25°C) and the same conditions of applied endotoxin, and then similarly the microparticles were isolated and collected, and reacted with AL. The results are shown in Fig. 4(b). As understood from the figure, the adsorption degree reached the maximum at about 1 hour of the reaction time. Although long adsorption reaction time was not tried, it is considered that the adsorption reaction may be performed for 20 to 30 minutes at room temperature from the results of Fig. 4(b) in consideration of the convenience of the measurement.

### <Example 3> (Example in which endotoxin-adsorption material previously adsorbed onto endotoxin is bound to microparticle)

In the description of Fig. 3 in Example 2, the silica microparticles bound with poly-L-lysine as the endotoxin-adsorption material as described in Preparation Example 4 were mixed with an endotoxin-containing solution, and centrifuged to precipitate the microparticles, and then the microparticles was re-suspended in an aqueous solution of a non-ionic surfactant Triton X-100, and this was mixed and reacted with AL reagent. On the other hand, in this Example, first, a water-soluble endotoxin adsorption material is mixed with a sample containing endotoxin, to adsorb endotoxin onto the endotoxin-adsorption material. Then, silica microparticles that are carrier microparticles are added to the mixed solution, whereby to bind the endotoxin-adsorption material in the state of being adsorbed with endotoxin onto the silica microparticles. According to this, it is possible to more easily prepare the endotoxin-adsorption microparticles.

### <Example 4> (Investigation of adsorption-dose reaction by coagulogen-pretreated type microparticle)

In Example, the microparticles itself (the case where the microparticles can adsorb both of endotoxin and coagulogen (bind to both)) and microparticles bound with the endotoxin-adsorbing adsorption material (the case where the adsorption material has affinity to endotoxin and coagulogen) were applied to the sample containing endotoxin. Then, the microparticles having adsorbed endotoxin and were reacted with AL at the time of measurement of endotoxin. On the other hand, in this Example, the microparticles bound with the endotoxin-adsorption material were further previously bound with coagulogen. This makes it possible to expect that endotoxin can be measured in a shorter time in comparison to the case where the microparticles are bound (adsorbed) to AL (coagulogen) for the first time at the time point where the microparticles are reacted with AL.

In other words, in the case where the microparticles adsorbed with endotoxin are reacted with AL, first, coagulogen quickly bind to the surface of the microparticle not adsorbed with endotoxin as described above. Then, endotoxin on the microparticle binds to C factor in AL, whereby to increase the activity of AL, and change coagulogen to coagulin, which induces the aggregation reaction involving the microparticles. In that case, 2-step actions occur in the case where the microparticles adsorbed with endotoxin are reacted with AL, and the aggregation of the microparticles gently proceeds as well, and thus accurate detection of the initiation time of the aggregation may become difficult.

In contrast, if the microparticles bound with the endotoxin-adsorption material are further previously bound with coagulogen, and the microparticles in this state are reacted with AL, coagulogen already bound to the microparticles rapidly changes to coagulin, which induces the aggregation reaction involving the microparticles. Accordingly, in comparison to the case where coagulogen is not previously bound to the microparticle, aggregation of the microparticles proceeds clearly and steeply, and thus it is possible to measure endotoxin more accurately in a short time.

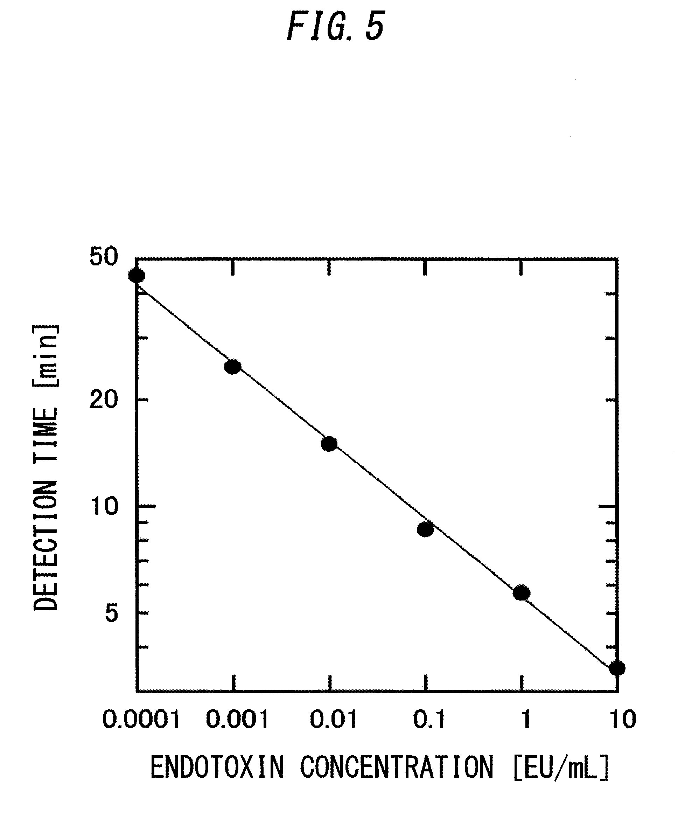

Herein, using the microparticles prepared by Preparation Example 5, it was investigated whether or not extremely low concentration of the endotoxin can be measured, which was extremely difficult to measure conventionally, or was not provided with a measurement method. Measurement of endotoxin in a broad range from an extremely low concentration such as 0.0001 EU/mL to 10 EU/mL was performed using the microparticles described in Preparation Example 3 in 1 mL of the liquid volume of the sample for 30 minutes of the reaction time at room temperature of the reaction temperature based on the results investigated in Example 2. In that case, it was possible to measure endotoxin in a broad range with very high linearity as shown in Fig. 5. It was possible to measure 0.0001 EU/mL of the endotoxin within 50 minutes. As described above, it was understood in this Example that the microparticles having adsorbed endotoxin keep coagulogen previously on the particles, and thus can be incorporated into the aggregate of AL at an early stage of the aggregation, to induce the aggregation reaction in a shorter time.

### <Example 5> (Concomitant use of coagulogen-bound microparticles)

By the way, endotoxin can be measured in a short time with the LAL-bound bead described in Patent Literature (JP-A No. 2009-150723) (hereinafter, referred to as the AL-bound bead.) as described above. By concomitantly using this AL-bound bead with the endotoxin-adsorption microparticles of the present invention, it may be possible to measure endotoxin in a further shorter time. In order to confirm this, 100 µL of the titania microparticles (PLK-P25) immobilized with poly-L-lysine of Preparation Example 6 was reacted with a sample containing endotoxin (1 mL). Then, the mixed solution was centrifuged and the microparticles were collected, and dispersed in Triton X-100 (0.02%, 50 µL). Then, the dispersion was reacted with 50 µL of an AL reagent (ES-II Multi reagent manufactured by Wako Pure Chemical Industries, Ltd.), to which one-tenth amount of the alumina microparticles (AL-ASFP) immobilized with coagulogen described in Preparation Example 7 was previously added.

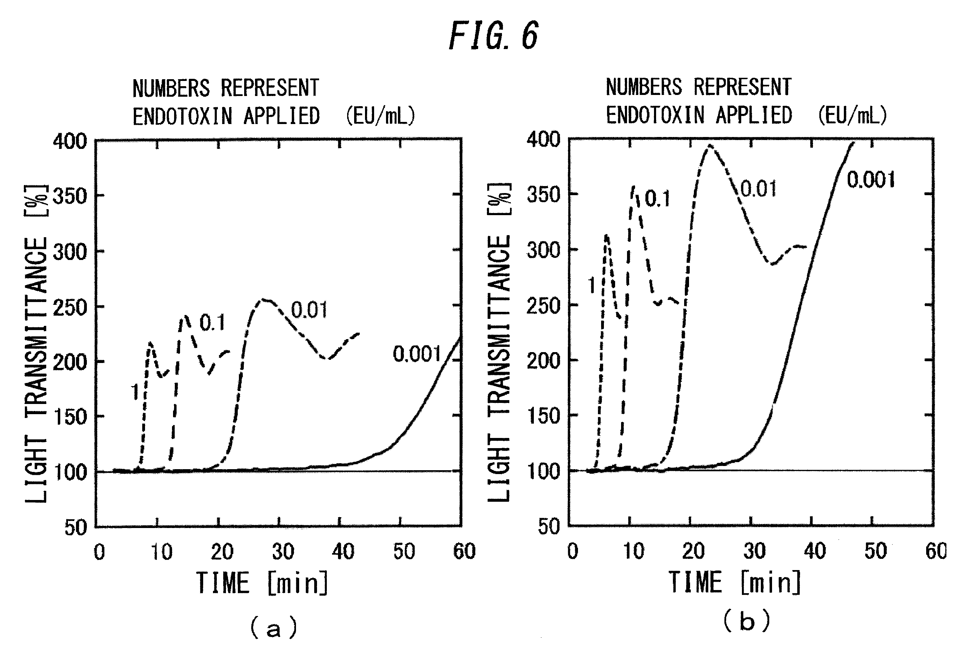

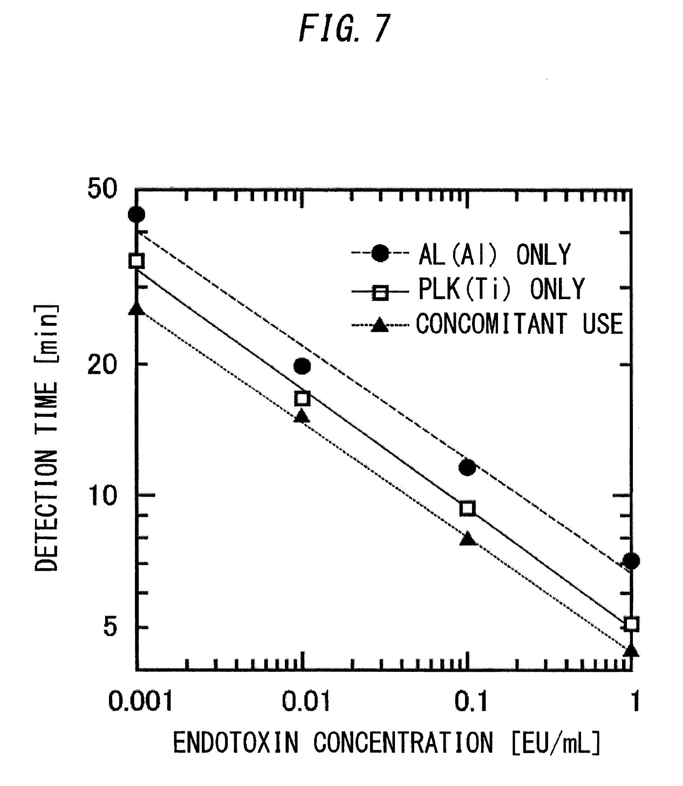

The reaction curve is shown in Figs. 6(a) and 6(b). The results of the conventional aggregation (reaction of 50 µL of the liquid, in which PLK-P25 is dispersed directly in each concentration of an aqueous solution of endotoxin, with 50 µL of AL) are shown in Fig. 6(a). The results of this Example are shown in Fig. 6(b). As understood from Figs. 6(a) and 6(b), concomitant use of the AL-bound bead and the endotoxin-adsorption microparticles of the present invention allowed to achieve significant shortening of the detection time. In addition, as shown in Fig. 7, it could be confirmed that concomitant use of the endotoxin-adsorption microparticles and the reagent in which AL was added to AL-ASFP, allowed shortening of the detection time in comparison to the case of single use in each concentration of endotoxin. As described above, concomitant use of the endotoxin-adsorption microparticles and the AL-bound bead allows short-time measurement with high sensitivity.

### <Example 6> (Measurement using AL interference substance)

An interference substance inhibiting or promoting the AL reaction happens to be contained in a sample for which measurement of endotoxin is performed. For example, serine protease cascade, which is very similar to AL, is contained in the blood. Particularly thrombin and anti-thrombin III in the blood directly act on the protease activity of AL and interferes with the AL reaction. Accordingly, in the case where endotoxin in the blood is measured, a heat-dilution method is used in which the blood plasma is diluted with a buffer containing a surfactant to 10 folds or so, and heated whereby to suppress the action of the interference substances. On the other hand, anti-thrombin III described above is used as an antithrombotic medicine for injection. In this case, anti-thrombin III binds to the core of the serine protease activity of thrombin and neutralizes the function of thrombin whereby to disturb promotion of the coagulating system.

This neutralization action also strongly acts on the serine protease of AL, and the AL reaction is completely suppressed if anti-thrombin exists. Thus, a sample containing anti-thrombin III is one of the samples for which the measurement of endotoxin is most difficult. In measuring endotoxin in a sample having interfering action on AL, the interfering action needs to be suppressed by sufficiently diluting the sample, and the like as described above. However, the interfering action of anti-thrombin cannot be suppressed unless anti-thrombin is highly diluted. As a result, endotoxin itself is diluted beyond the measurable range, and difficult to measure with a conventional measurement method. In the present invention, it is possible to adsorb and concentrate endotoxin from the sample, and at the same time, wash the microparticles after the reaction as necessary whereby to remove the interference substance. It was considered in this Example that if endotoxin of a sample containing anti-thrombin III, which is one of the samples most difficult to measure, can be measured, other kinds of samples can be also measured by the present invention.

Anti-thrombin III (Neuart, manufactured by Mitsubishi Tanabe Pharma, 50 U/mL) was diluted by 10 folds with distilled water for injection, and endotoxin was added to this in 0.01 EU/mL of the concentration. To this solution (1 mL), 100 µL of a dispersion liquid of the titania microparticles described in Preparation Example 2 (P25, 0.3 mg/mL, 0.2% Triton X-100) or a dispersion liquid of the titania microparticles bound with poly-L-lysine of Preparation Example 6 (PLK-P25, 0.3 mg/mL, 0.2% Triton X-100) was added. Then, the mixture was reacted for 30 minutes at room temperature whereby to adsorb endotoxin onto each of the microparticles.

Each of the microparticles after the adsorption was centrifuged to isolate and collect the microparticles. Then, first, a dispersion in which respective precipitate was dispersed in 0.02% aqueous solution of Triton X-100 (50 µL) (ATIII-P25-not washed, and ATIII-PLK-P25-not washed) was prepared. In addition, a dispersion in which respective precipitate was dispersed in 0.02% aqueous solution of Triton X-100 (1 mL), was centrifuged again and precipitated, and the supernatant was removed, and then the precipitate was dispersed again in the same aqueous solution. Such operations were repeated three times to prepare the washed microparticles (ATIII-P25-washed, and ATIII-PLK-P25-washed).

In addition, 100 µL of a dispersion liquid of the titania microparticles described in Preparation Example 2 (P25, 0.3 mg/mL, 0.2% Triton X-100) or a dispersion liquid of the titania microparticles bound with poly-L-lysine of Preparation Example 6 (PLK-P25, 0.3 mg/mL, 0.2% Triton X-100) was added respectively to an aqueous solution not containing ATIII and containing 0.01 EU/mL of endotoxin, and the mixture was reacted for 30 minutes at room temperature whereby to adsorb endotoxin onto each of the microparticles. Then, the microparticles were centrifuged to isolate and collect the microparticles, and a dispersion in which respective precipitate was dispersed in 0.02% aqueous solution of Triton X-100 (50 µL) (P25-Control, and PLK-P25-Control) was prepared. These microparticles were mixed with 50 µL of AL (ES-II Multi reagent manufactured by Wako Pure Chemical Industries, Ltd.) whereby to induce the aggregation reaction.

The results are shown in Table 1. With the samples not washed with ATIII, i.e., ATIII-P25-not washed and ATIII-PLK-P25-not washed, the aggregation was not induced within 100 minutes of the measurement time when the concentration measured with the control, i.e., P25-Control or PLK-P25-Control was assumed 100%. On the other hand, with the samples washed after the reaction with a sample containing ATIII, i.e., ATIII-P25-washed and ATIII-PLK-P25-washed, the aggregation was induced. However, with ATIII-P25-washed in which the ceramic microparticles themselves (P25) were used as the adsorption material, the collection rate decreased. On the other hand, with ATIII-PLK-P25-washed in which the microparticles bound with poly-L-lysine (PLK-P25) were used, the collection rate did not change.

**[Table 1]**

| | | Adsorbing agent | |
|---|---|---|---|
| | | P25 (Titania) | PLK-P25 |
| Addition Collection rate | Control | 100.0% | 100.0% |
| | ATIII-washed | 36.5% | 107.9% |
| | AT III-not washed | 0.2% or less | 0.2% or less |

From this, it was understood that even in a sample containing anti-thrombin III which is most difficult to measure, it is possible to remove the interfering action by washing the microparticles after the adsorption of endotoxin by using the endotoxin-adsorptive aggregation microparticles in the present invention. In addition, it was understood that the microparticles to be used are desirably microparticles bound with an adsorption material having high selectivity for endotoxin (which should not limited to PLK-P25 exemplified in this Example). The reason for the decrease of the collection rate in the case where P25, which has low selectivity for the adsorption, is used, is considered that anti-thrombin is non-specifically adsorbed onto P25 since anti-thrombin III is contained in a high concentration, and thus adsorption of endotoxin is subjected to competitive inhibition.

By combining the endotoxin-adsorptive aggregation microparticles of the present invention with the washing operation, it was possible to perform endotoxin measurement without being affected by an interference substance. In this case, the microparticles are desirably as minute as possible within a range where the microparticles are easily precipitated. This is because if the microparticle is large, the specific surface area is small and thus the adsorption site of endotoxin is small, and thus adsorption efficiency of endotoxin decreases. In addition, this is because if the microparticle is a silica gel having 50 µm or so of the particle diameter as described in Patent Literature 1, the particle diameter is large and thus the liquid is kept in the inside of the particle, and the interference substance such as anti-thrombin III kept in the inside of the particle cannot be easily removed even with the washing operation. The microparticle of the present invention has 10 µm or so of the primary particle diameter at most, and thus the liquid kept in the inside of the particle is small and easily washed.

On the other hand, it is considered that measurement of endotoxin in the blood is easier than measurement of endotoxin in anti-thrombin III. This is because anti-thrombin III preparation is manufactured by concentrating anti-thrombin III in the blood, and thus the inhibition action of anti-thrombin III to AL is extremely larger in comparison to the inhibition action of the blood. Herein, it is estimated that in the case where endotoxin in the blood is measured, it is also possible to easily measure endotoxin without being affected by the interference substance in the blood by combining the endotoxin-adsorptive aggregation microparticles of the present invention with the washing operation, although not shown in Examples.

### <Example 7> (Endotoxin-concentration action with various endotoxin-adsorption material)

As the adsorption material for endotoxin, a polymer containing a primary or secondary amine in the side chain is frequently used. In addition, a peptide selected from a random peptide library also starts from a first amino acid that is an amino acid having amine in the side chain of any one of arginine, lysine and histidine. One amino group of an amino acid is used in the peptide bond, and thus it was examined if a polyamino acid homopolymer having a residual amino group in the side chain, which is poly-L-lysine, and in addition, poly-L-arginine, poly-L-histidine or poly-L-ornithine, has adsorption concentration effect. In addition, polyethylene imine, which is known to have high content of the amino group, was examined similarly.

As each of the substances, an amino acid homopolymer manufactured by Sigma Aldrich and polyethylene imine manufactured by Wako Pure Chemical Industries, Ltd. were used. Each of the substances or polyethylene imine was diluted with distilled water for injection to 0.1% (1% of polyethylene imine), and mixed with 10 mg/mL of titania nanoparticle (AEROXIDE P-25) dispersion liquid in the volume ratio of 9 : 1, and reacted for 1 hour at room temperature. Next, the reaction liquid was centrifuged to remove the supernatant, and the residue was re-suspended in distilled water for injection. Washing by this method was performed three times in total, to remove the unreacted adsorption material.

The titania nanoparticles were dispersed in 0.02% aqueous solution of Triton X-100 in 0.2 mg/mL in conversion to the initial titania nanoparticle. 0.1 mL of the microparticle was added to 1 mL of an aqueous solution of 0.01 EU/mL endotoxin and reacted at room temperature for 20 minutes, and then centrifuged, whereby to remove the supernatant, and dispersed in 50 µL of 0.02% aqueous solution of Triton X-100. This was mixed with 50 µL of AL, and the aggregation reaction was examined with a device for the stirring turbidimetric method (EX-100). As a result, it was confirmed that endotoxin is adsorbed and concentrated on the microparticle, and the measurement time is drastically shortened in comparison to the case where a sample itself having the same concentration of endotoxin is applied in any adsorbing agent containing an amino group.

### <Example 8> (Measurement of β glucan)

Concentration of endotoxin by the microparticles and aggregation of the microparticles have been explained until Example 7. In Example 8, the aggregation reaction by β glucan will be explained. Evaluation was performed using curdlan (manufactured by Wako Pure Chemical Industries, Ltd.) that is one of linear β-1,3 glucans. It is known that an AL reagent originally reacts with endotoxin or β glucan. It is known that an endotoxin-specific reagent has a mechanism of inhibiting the actions of glucan by adding analogs of β glucan in a large amount to the reagent, or removes a protein to which β glucan is bound. In this Example, Limulus HS-T (Wako Pure Chemical Industries, Ltd.), which is an AL reagent not specific for endotoxin, was used.

Curdlan was dissolved in distilled water for injection, and 10-fold dilution series from 10 ng/mL to 1 pg/mL of the concentration were prepared. These dilution series (50 µL) and HS-T (50 µL) were put into the vessel made of glass manufactured in Preparation Example 1, and mixed, and the aggregation reaction was measured with EX-100 (conventional method). On the other hand, a method of adsorbing and concentrating curdlan to the titania microparticles bound with polylysine manufactured in Preparation Example 6, and then reacting it with AL was performed as described below. In other words, 100 µL of a dispersion liquid of the microparticles of Preparation Example 6 was added to 1 mL aqueous solution of curdlan, and shaked and stirred for 10 minutes With a Vortex mixer whereby to adsorb curdlan onto the microparticles.

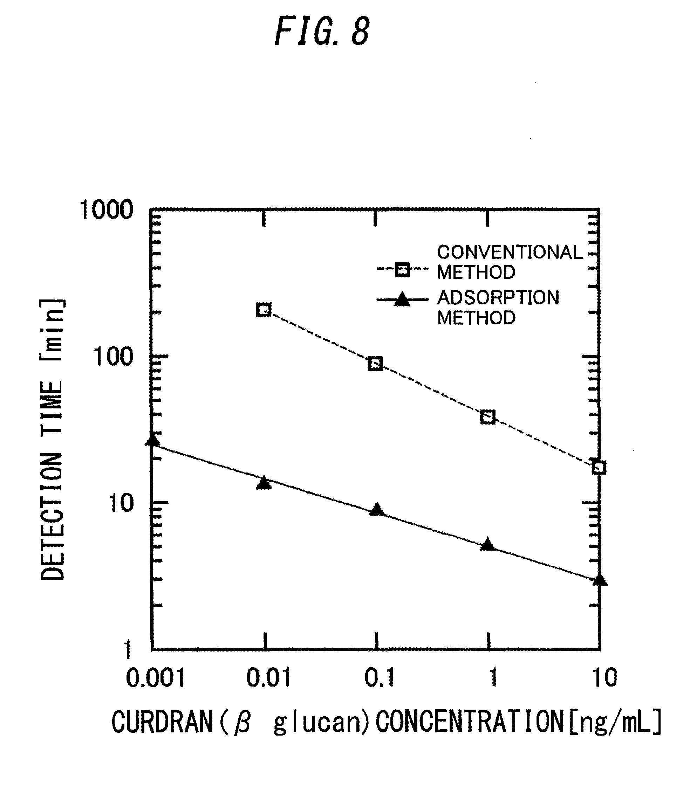

Then, the microparticles were centrifuged to precipitate the microparticles, and this was dispersed in distilled water for injection containing 0.02% Triton X-100 (50 µL). The dispersion liquid of the microparticles was mixed and reacted with HS-T (50 µL) in the glass vessel manufactured in Preparation Example 1, and the aggregation reaction was measured with EX-100 (adsorption method: the present invention). The results are shown in Fig. 8 in which the concentration of curdlan at the horizontal axis and the detection time of curdlan at the vertical axis were plotted with a logarithm for both of them. As shown in the figure, any of the methods showed high linearity in broad concentrations of curdlan. However, the detection required very long time in the case where curdlan was measured with the conventional method. On the other hand, with the present invention, it was possible to drastically shorten the detection time, and detect curdlan practically within 30 minutes or less of the time even at a low concentration (0.001 ng/mL).

The polylysine used as the adsorbing agent herein has not been reported as a selective adsorbing agent for glucan. However, the mechanism of adsorbing glucan onto the microparticles by polylysine will be discussed below. The glucan usually forms advanced hydrogen bond between the alcohols of the adjoining chains, and thus has a low solubility in water. However, it is known that alkali treatment at this time cleaves the hydrogen bond. Accordingly, a mechanism is considered in which the amino groups of polylysine having very high density of the amino groups act as alkali, and cleave the hydrogen bond of the glucan, and form a hydrogen bond with the alcohol of the glucan whereby to adsorb the glucan. In addition, considered are a mechanism in which polylysine on the microparticle catches glucan by stirring and the like, whereby to adsorb and immobilize the glucan onto the microparticle since both of polylysine and glucan are polymers, and the like. However, the details are unknown at present. In any case, the fact that polylysine can adsorb not only endotoxin but also glucan onto the microparticles, is desirable from the view point that the same adsorption material can be used in measuring any of the substances.

Next, an embodiment for implementing the present invention with respect to a medical device will be specifically explained using the figures.

As the microparticle in the present embodiment, also considered may be (1) inorganic microparticles having low selectivity for endotoxin, but being capable of adsorbing endotoxin and coagulogen in AL, and (2) inorganic carrier microparticles bound with an organic adsorption material having high selectivity and affinity for endotoxin. In the former case, the selectivity for endotoxin is low, and thus is good as a material for the medical device, but the function as an endotoxin-adsorption material decreases in the case where the ingredients contained in the material of medical device is more strongly bound with endotoxin than the inorganic microparticles, or in the case where the ingredients of the material of the medical device bind to inorganic microparticles. On the other hand, the latter has high binding specificity to endotoxin, and thus can sufficiently exert the ability as the adsorption material whereby to extract endotoxin from the medical device and adsorb it on the microparticle, and thus is expected to exert high performance.

In determining the endotoxin concentration using the microparticles of the present invention, a dispersion liquid containing the prepared microparticles is used as an extraction liquid, and this is suitably brought into contact with a medical device. Then, endotoxin is extracted from the medical device for an appropriate time and at the same time adsorbed onto the microparticles, and then microparticles are precipitated by a method such as centrifugal isolation. The extraction operation largely varies depending on the structure or the material of the medical device to be evaluated, and thus optimal extraction method may be investigated in advance and suitably determined. For example, in the case where the subject is a filtration filter (filter having coarse meshes), an extraction liquid containing the endotoxin-adsorbing microparticles may be passed through the device several times as necessary, and endotoxin adhered may be extracted and adsorbed onto the microparticles.

In addition, in the case where the subject is a filtration filter having fine meshes, and the microparticles cannot pass the filter, or in the case where the subject has a complicated structure and the extraction liquid is difficult to pass, measures such as putting the endotoxin-adsorbing microparticle into the inside of the device, or dipping the device in the extraction liquid may be performed. Then, treatments such as shaking and ultrasonic wave treatment may be performed as necessary to enhance the extraction efficiency of endotoxin from the device. In addition, an aqueous solution of an endotoxin-adsorptive organic material having high affinity to endotoxin may be used as the extraction liquid without using the extraction liquid in which the endotoxin-adsorbing microparticles are dispersed in the extraction of endotoxin from the medical device. In this case, after extraction of endotoxin, these endotoxin-adsorptive organic materials may be mixed with bindable carrier microparticles whereby to bind the organic material adsorbed with endotoxin to the carrier microparticles.

In addition, the case may be assumed where ingredients eluting from the medical device exist, and they have interfering action with AL. In this case, the microparticles adsorbed with endotoxin are washed with appropriate water (injection water and the like) not containing endotoxin. Specifically, the washing is performed by performing the processes such as re-suspending the microparticles adsorbed with endotoxin in such water, and precipitating the microparticles again by centrifugal isolation and the like, and removing the supernatant. It is natural that the washing effect is improved by repeating the processes multiple times as necessary.

In addition, by adding an ingredient having a surfactant effect to the dispersion liquid of the microparticles mixed with the sample, it is possible to stabilize the microparticles at the time of the endotoxin-adsorption reaction, and disperse endotoxin by the surfactant action whereby to effectively adsorb endotoxin to the microparticles. In addition, if the microparticles themselves, or the endotoxin-adsorption material bound to the microparticles rapidly bind to coagulogen in AL when the microparticles adsorbed with endotoxin are mixed with an AL reagent, an aggregation reaction having nothing to do with the aggregation reaction by endotoxin may be induced. Also from the meaning of suppressing such rapid binding, it is desirable to add an ingredient having surfactant action to the dispersion liquid in which the precipitated microparticles are re-suspended.

In Examples described below, methods for measuring endotoxin by measuring the aggregation reaction with a stirring turbidimetric measurement device (EX-100, manufactured by Kowa Company Ltd.) will be exemplified. However, a time-resolved particle size distribution device for analysis of EX-300 having a stirring system and using laser light scattering (manufactured by Kowa Company, Ltd.) may be used since the aggregation reaction of a mixture of the microparticles and AL can be measured. In addition, the stirring is not necessarily regularly performed since the object of the stirring of the sample is induction of the aggregation, the light transmittance or the light scattering may be measured using a micro plate reader having a stirring function of the sample. In addition, ultrasonic wave may be used in the case where a very small quantity of the sample is stirred.

### <Preparation Example 8> (Endotoxin-free glass vessel)

A special vessel made of glass having 6 mm ϕ and 50 mm length and provided with a stirring bar made of stainless steel for stirring the sample (0.75 mm ϕ, 3.5 mm length) was also used in the light transmittance measurement method in the case where the present invention is implemented for a medical device. On the other hand, a special vessel made of glass having 7 mm ϕ and 50 mm length and provided with a stirring bar made of stainless steel for stirring the sample (1 mm ϕ, 5 mm length) was used in the laser light scattering particle counting method. The openings of these glass vessels were covered with an aluminum foil. Further, those packaged with an aluminum foil in subdivision to 20 pieces were put into a hot-air treatment can made of iron, and the mixture was heat-treated for 3 hours at 250°C, whereby to thermally decompose endotoxin.

### <Preparation Example 9> (Inorganic microparticle having endotoxin adsorption ability)

Titania microparticles AEROXIDE P25 manufactured by Nippon Aerosil Co., Ltd. (primary particle diameter: 20 nm) were subdivided into glass tubes. Then, the openings of the glass tubes were covered with an aluminum foil. Further, several pieces were collected and put into a glass beaker, and the openings were further covered with an aluminum foil, and hot-air treated for 30 minutes at 250°C whereby to completely inactivate endotoxin which is a risk of causing contamination. This was dispersed in a dispersion liquid to the predetermined concentration whereby to prepare aggregation microparticles having no endotoxin selectivity but having adsorption ability.

### <Preparation Example 10> (Titania microparticle immobilized with organic material having endotoxin adsorption ability)

The titania microparticles AEROXIDE P25 manufactured by Nippon Aerosil Co., Ltd. prepared in Preparation Example 9 were dispersed in an aqueous solution of 0.1% poly-L-lysine (manufactured by Sigma, Aldrich) in 10 mg/mL of the concentration. The dispersion liquid was slowly stirred at room temperature for 2 hours whereby to adsorb poly-L-lysine onto the titania microparticle. Then, the dispersion liquid was centrifuged and the supernatant was removed, and the residue was washed three times with distilled water for injection not containing endotoxin. Finally, the residue was dispersed in a non-ionic surfactant Triton X-100 (0.2%) in 0.2 mg/mL of the converted concentration of the beads of the material whereby to prepare aggregation microparticles having selective endotoxin-adsorption ability (PLK-P25).

### <Preparation Example 11> (Preparation of tube made of resin adsorbed with endotoxin)

An aqueous solution of endotoxin (10 EU/mL, 1 mL) was put into an autoclave-treated Eppendorf tube of 1.5 mL volume, and stood one day, to adsorb and bind a portion of endotoxin in the solution onto the interior wall of the tube. The solution in the tube was all discharged, and 1 mL of distilled water for injection was put into, and stirring was performed for 10 seconds at the maximum rotation speed with a Vortex mixer (TUBE MIXER TRIO HM-1F manufactured by AS ONE Corporation). Then, the solution in the tube was all discharged. This operation was performed twice in total, and the remaining solution of endotoxin not adsorbed onto the interior wall within the tube was removed. By this, an Eppendorf tube in which endotoxin was physically adsorbed onto the interior wall, was prepared. Furthermore, it is known that an Eppendorf tube is made of polypropylene, and polypropylene or polyethylene is a hydrophobic material and therefore endotoxin is adsorbed on an Eppendorf tube (for example, see Non-Patent Literatures 2 and 3.).

Furthermore, also in Preparation Examples, poly-L-lysine was used as the endotoxin-adsorption material. As polylysine, there are α poly-D/L-lysine, an original polypeptide in which an α-position amino group and a carboxyl group are linked by an amide bond (= peptide bond), and ε poly-L-lysine in which an ε-position amino group and a carboxyl group are linked by an amide bond (not a peptide bond in the narrow sense). Since the L form is easily decomposed by enzymes contained in a cell, the D form, which is hardly decomposed, may be used as necessary. Although the present embodiment using poly-L-lysine will be explained, either poly-L-lysine or poly-D-lysine may be used as the adsorption material.

Next, endotoxin measurements with the microparticles having the endotoxin-adsorption ability with respect to a sample containing thin endotoxin and a sample having endotoxin interfering action will be explained.

### <Example 9> (Endotoxin extraction and endotoxin-concentration action by endotoxin-adsorptive microparticles and measurement by aggregation reaction)

A method for measuring endotoxin using AL is a calibration curve method in which a calibration curve is prepared on the basis of measurements of endotoxin dilution series of known concentrations. Accordingly, it is necessary to prepare respective calibration curves in the case where the endotoxin-adsorptive microparticles are not used, and in the case where the endotoxin-adsorptive microparticles are used. In the case where the microparticles were not used, 50 µL of an aqueous solution of the endotoxin dilution series (0.1, 0.01, 0.001 EU/mL) and 50 µL of the AL reagent were put into the tube of Preparation Example 8 and the aggregation reaction was measured with a stirring turbidimetric measurement device (EX-100, manufactured by Kowa Company Ltd.).

In addition, in the case where the microparticles were used, 100 µL of a dispersion liquid of the endotoxin-adsorptive microparticles of Preparation Example 10 was put into 1 mL of an aqueous solution of the same endotoxin dilution series in the tube of Preparation Example 8 described above. Then, the tube was immobilized on a special attachment capable of keeping and stirring multiple Eppendorf tubes. Then, the scale of the stirring rotation number was set to 3.25, and the tube was stirred for 10 minutes whereby to adsorb endotoxin in the dilution series onto the endotoxin-adsorptive microparticles.

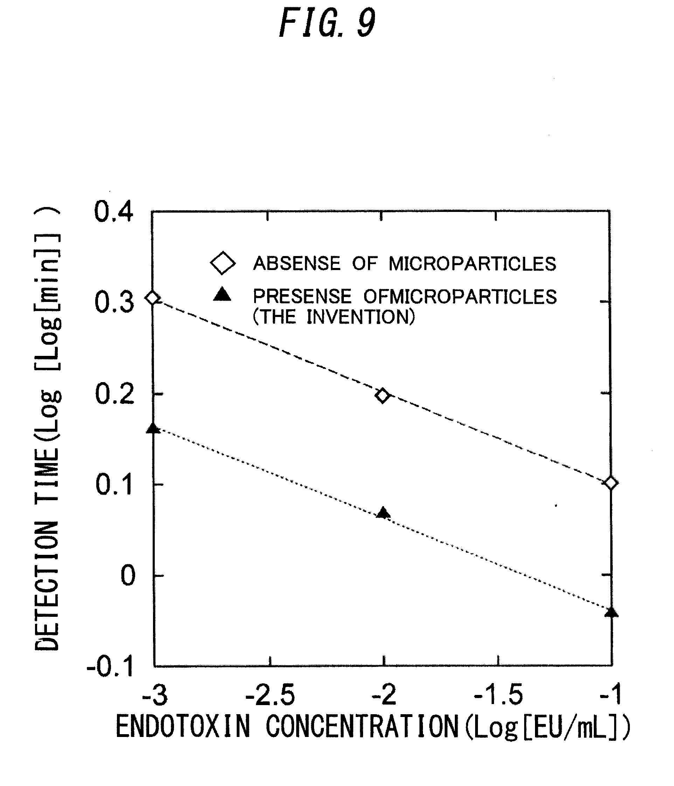

Subsequently, the tube was centrifuged at 11,000 x g of the centrifuge acceleration for 2 minutes to precipitate the microparticles, and the supernatant was removed with a micropipetter carefully without sucking the precipitate. The precipitated microparticles were dispersed in 60 µL of 0.02% aqueous solution of Triton X-100 added with pipetting, and 50 µL of the dispersion was mixed with 50 µL of AL in the glass vessel of Preparation Example 8 and the measurement was performed with EX-100. The time point where the light transmittance reached 95% in the case where the microparticles were not contained, and the time point where the light transmittance reached 105% in the case where the microparticles were contained, were assumed as the detection time of endotoxin when the initial light transmittance was taken as 100% in detecting the aggregation of the dilution series samples of the respective endotoxin concentrations. The results of the measurements are shown in Table 2 and Fig. 9.

**[Table 2]**

| Concentration of dilution series(EU/mL) | Detection time of endotoxin (minute) | |
|---|---|---|
| | Microparticles not contained | Microparticles contained |
| 0.1 | 18.3 | 8.1 |
| 0.01 | 37.6 | 14.7 |
| 0.001 | 104.5 | 28.2 |

As understood from Table 2, the detection time was drastically shortened in the case where the adsorption microparticles were contained in comparison to the case where the adsorption microparticles were not contained. This is due to the synergistic effect of the point that endotoxin can be concentrated by using the microparticle method and the point that measurable generation of the aggregation mass is accelerated by the reaction of endotoxin adsorbed on the microparticle with AL. Then, the concentrations of endotoxin were on the line as shown in Fig. 9 when the logarithmic values of known endotoxin concentrations of these dilution series samples for the calibration curve (the horizontal axis) and the two-time logarithmic values of the detection time (the vertical axis) are plotted.

Next, the endotoxin extraction effect of the endotoxin-adsorptive microparticles of the present invention from a medical device and the measurement-shortening effect were evaluated. 1 mL of water for injection was freshly put into the Eppendorf tube prepared in Preparation Example 11, and subsequently the dispersion liquid of the microparticles prepared in Preparation Example 9 or Preparation Example 10 was added in 0 (in the case where the dispersion liquid was not put) or 100 µL. The Eppendorf tube was immobilized on a Vortex mixer, and was stirred for 10 minutes under the similar conditions as described above, in other words, setting the scale of the rotation speed to 3.25.

In the condition where 100 µL of the dispersion liquid of the microparticles of Preparation Example 9 was put into, the tube was then centrifuged with a centrifugal separator at 11,000 x g for 2 minutes to precipitate the microparticles. Similarly as described above, the supernatant was removed and 50 µL of a dispersion liquid of the microparticles dispersed in 60 µL of 0.02% aqueous solution of Triton X-100 and 50 µL of the AL reagent were put into the glass vessel of Preparation Example 8 and the aggregation reaction was measured with EX-100. In the case where the microparticles were not put into, the extracted water after the extraction reaction was taken by 50 µL, and this was put along with 50 µL of the AL reagent into the glass vessel of Preparation Example 8 and the aggregation reaction was measured with EX-100 similarly as described above.

The detection time of endotoxin in the case where the microparticles were not put into, was 118.5 minutes. This was beyond the detection time at 0.001 EU/mL which was the lowest concentration of the dilution series of Table 2, and thus it was strictly not possible to quantify endotoxin at the measurement sensitivity of 0.001 EU/mL or less (0.00074 EU/mL when calculated with extrapolation of the calibration curve). On the other hand, the detection time was 13.9 minutes in the case where the microparticles of Preparation Example 9 were used, and the detection time was 14.3 minutes in the case where the microparticles of Preparation Example 10 were used. These are respectively detection times located between the two samples having thick concentration of the dilution series, and corresponded to 0.0110 EU/mL in the case where the microparticles of Preparation Example 9 were used, and 0.0099 EU/mL in the case where the microparticles of Preparation Example 10 were used when the concentration was calculated according to the calibration curve.

In this Example, the degrees of the effect were equal in those prepared in Preparation Example 9 where the endotoxin-adsorptive organic compound was not bound onto the microparticles, and in those prepared in Preparation Example 10 where the endotoxin-adsorptive organic compound was bound onto the microparticles. However, it is assumed that in microparticles not bound with an organic compound having high endotoxin selectivity, the extraction and adsorption of endotoxin to the microparticles from the device may not be sufficient depending on the material of the device. By using properly the presence or absence of the endotoxin-adsorptive organic compound depending on the material of the device, it is possible to obtain good results with a device of any material. As described above, with the present invention for achieving measurement of the endotoxin concentration by extracting a very small quantity of endotoxin adhered to a device and adsorbing it on the microparticle and concentrating the microparticles by centrifugal isolation and the like, it is possible to measure the concentration of endotoxin adhered to the device simply in a short time.

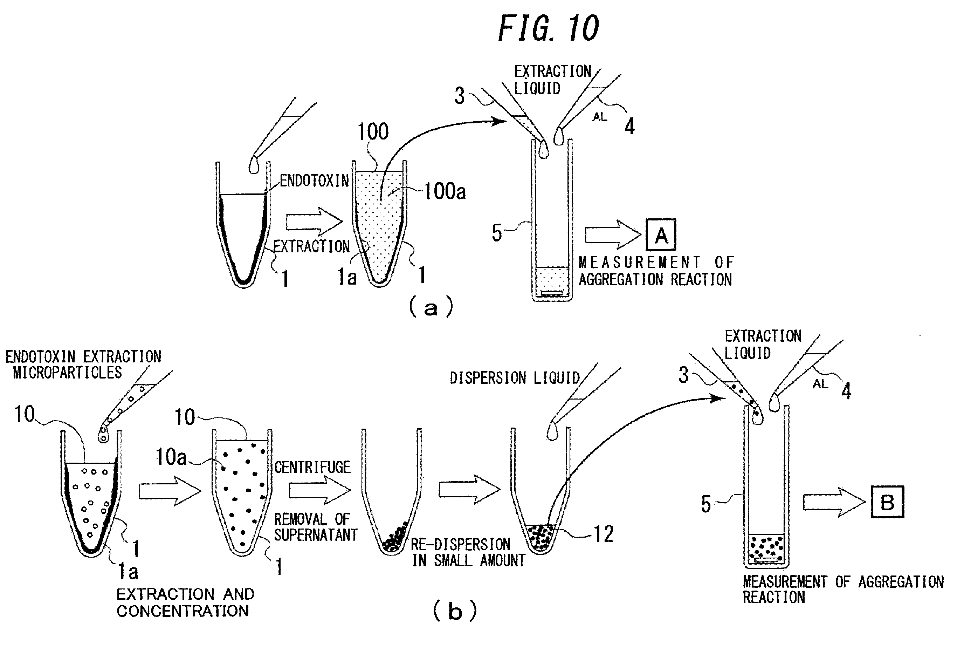

The outline of the present invention can be shown in Figs. 10(a) and 10(b). The flow of the measurement shown in Fig. 10(a) explains the case where the endotoxin-adsorptive microparticles were not used, and the flow of the measurement shown in Fig. 10(b) explains the case where the endotoxin-adsorptive microparticles were used. In Fig. 10(a), 1 mL of water for injection is added to an Eppendorf tube 1 in the state of being adhered with endotoxin. In that case, endotoxin 1a adhered to the Eppendorf tube 1 is dispersed in water for injection 100. However, in this case, endotoxin 100a dispersed in the water for injection 100 cannot be concentrated, and thus the ratio of those dispersed in the water for injection among the endotoxin 1a adhered to the Eppendorf tube 1 is limited. In addition, the endotoxin concentration in the water for injection 100 in which endotoxin is dispersed, is also low.

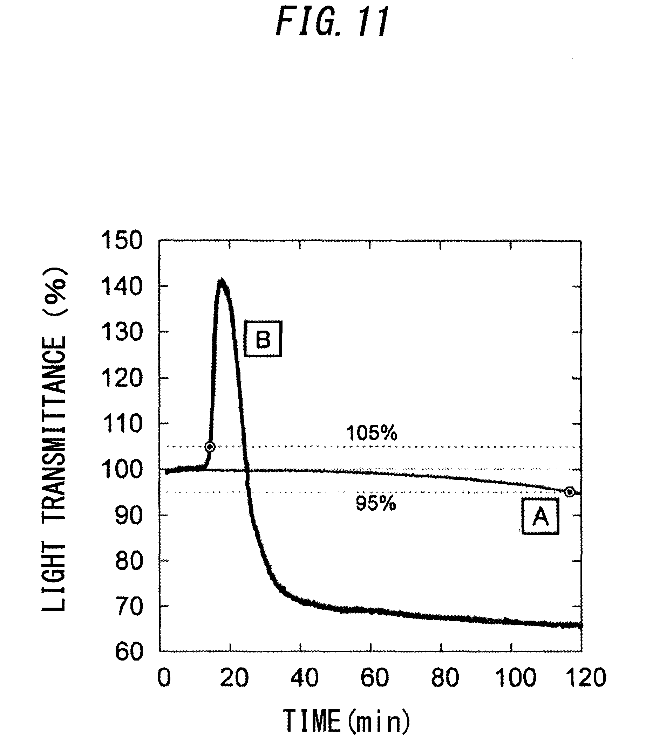

Next, 50 µL of the water for injection 100 in which endotoxin 100a is dispersed, is mixed with 50 µL of the AL reagent in a glass vessel 5 using a micropipetter 3 or 4, and the aggregation reaction is measured. The change of the light transmittance obtained as a result thereof is shown with the curve A of the graph shown in Fig. 11.

Next, in the flow of Fig. 10(b), 1 mL of water for injection is put into an Eppendorf tube 1 in the state of being adsorbed with endotoxin, and further 100 µL of a microparticle dispersion liquid in which endotoxin-adsorptive microparticles are dispersed is added to prepare an extraction liquid 10. In that case, endotoxin 1a adhered to the Eppendorf tube 1 is adsorbed onto the endotoxin-adsorptive microparticles and dispersed in the extraction liquid 10. At this time, the endotoxin dispersed in the extraction liquid 10 is preferentially adsorbed onto the endotoxin-adsorptive microparticles, and thus more portions of endotoxin adhered to the Eppendorf tube 1 move to the surface of the endotoxin-adsorptive microparticles 10a from the Eppendorf tube 1.

Next, the extraction liquid 10 in which the microparticles adsorbed with endotoxin 10a are dispersed, is centrifuged, and the supernatant is removed, whereby to isolate the microparticles adsorbed with endotoxin 10a. This allows the endotoxin in the extraction liquid 10 to be a concentrated form in the isolated microparticles 10a. This is re-dispersed in 60 µL of 0.02% aqueous solution of Triton X-100. Then, 50 µL of the dispersion liquid 12 and 50 µL of the AL reagent are mixed in a glass vessel 5 using a micropipetter 3 or 4, and the aggregation reaction is measured. The change of the light transmittance obtained as a result thereof is shown with the curve B of the graph shown in Fig. 11.

From the curve A in Fig. 11, it is understood that the light transmittance (%) of a mixed solution of the water for injection 100 in which endotoxin was dispersed, and the AL reagent slowly decreases by the aggregation reaction based on the reaction between endotoxin and AL. On the other hand, in the curve B, at the early stage, the microparticles adsorbed with endotoxin 10a are dispersed in a mixed solution of the dispersion liquid 12 and the AL reagent, and the mixed solution is in the state of being turbid, and the light transmittance (%) rapidly increases by the aggregation of the microparticles 10a with each other. As a result thereof, it is understood that the detection time (time taken for 5% change from the early stage in the graph) is drastically shortened in the curve B in comparison to the curve A.

Furthermore, the origin of the hemocyte extract of limulus (hereinafter, also referred to as "AL: Amoebocyte lysate".) does not matter in the present invention (LAL (Limulus Amebocyte Lysate) derived from American Limulus, TAL (Tachypleus Amebocyte Lysate) derived from Asian Limulus, and the like).

### Reference Signs List

- 1: Eppendorf tube
- 1a: Endotoxin
- 3: Micropipetter
- 4: Micropipetter
- 5: Glass vessel
- 10: Extraction solution
- 10a: Microparticles adsorbed with endotoxin
- 12: Dispersion liquid
- 100: Water for injection
- 100a: Endotoxin

## Claims

1. A method for measuring a physiologically active substance of biological origin by producing a mixed solution of an AL reagent containing AL which is a hemocyte extract of limulus and a sample containing a predetermined physiologically active substance of biological origin, and detecting protein aggregation or gelation induced by a reaction between AL and the physiologically active substance in the mixed solution with an optical technique while stirring the mixed solution, whereby to detect the physiologically active substance in the sample or measure the concentration of the physiologically active substance, which is **characterized by**
dispersing microparticles capable of adsorbing the physiologically active substance on the surface in the sample whereby to adsorb the physiologically active substance on the surface of the microparticle, and then isolating the microparticles from the sample,
producing a mixed solution of the AL reagent and the microparticle adsorbed with the physiologically active substance on the surface, and
detecting aggregation or gelation of the microparticles in the mixed solution of the AL reagent and the microparticles with an optical technique.

2. The method for measuring a physiologically active substance of biological origin according to claim 1, which is characterized that the sample containing a predetermined physiologically active substance of biological origin is an extraction liquid containing a physiologically active substance of biological origin extracted from a medical device.

3. The method for measuring a physiologically active substance of biological origin according to claim 1 or 2, which is characterized that the microparticle comprises one or multiple materials selected from PyroSep, polymixin B, polylysine, polyornithine, polyarginine, polyhistidine, aminosilane, chitosan, an anti-endotoxin antibody, an anti-endotoxin aptamer, a material in a random peptide library, a metal oxide such as alumina, titania, silica, zirconia and hydroxyapatite, and a natural or synthetic mineral such as kaolin, montmorillonite, manganese oxide and mica.

4. The method for measuring a physiologically active substance of biological origin according to claim 1 or 2, which is characterized that the microparticle is formed by binding an adsorption material selectively adsorbing the physiologically active substance onto the surface of the carrier microparticles.

5. The method for measuring a physiologically active substance of biological origin according to claim 4, which is characterized that the adsorption material is one or multiple materials selected from PyroSep, polymixin B, polylysine, polyornithine, polyarginine, polyhistidine, aminosilane, chitosan, an anti-endotoxin antibody, an anti-endotoxin aptamer and a material in a random peptide library.

6. The method for measuring a physiologically active substance of biological origin according to claim 4 or 5, which is characterized that the carrier microparticle comprises one or multiple materials selected from polystyrene latex, polyethylene, nylon, cellulose, agarose, polyvinyl alcohol, an acrylic resin, a metal oxide such as alumina, titania, silica, zirconia and hydroxyapatite, and a natural or synthetic mineral such as kaolin, montmorillonite, manganese oxide and mica.

7. The method for measuring a physiologically active substance of biological origin according to any one of claims 1 to 6, which is characterized that the diameter of the microparticle is 5 nm or more and 50 µm or less.

8. The method for measuring a physiologically active substance of biological origin according to any one of claims 1 to 7, which is characterized that a surfactant is added to the microparticles.

9. The method for measuring a physiologically active substance of biological origin according to claim 4, which is characterized that the microparticle is formed to be covered with the adsorption material on the whole surface.

10. The method for measuring a physiologically active substance of biological origin according to any one of claims 1 to 9, which is characterized that the microparticle comprises a material having an affinity to coagulogen in the AL reagent.

11. The method for measuring a physiologically active substance of biological origin according to any one of claims 1 to 10, which is characterized that the microparticle is formed to contain a material having an affinity to coagulogen, and is previously bound with coagulogen.

12. The method for measuring a physiologically active substance of biological origin according to any one of claims 1 to 11, which is characterized that in the case where an interference substance affecting the reaction between the AL and the physiologically active substance is contained in the sample, the microparticles after being isolated from the sample are washed whereby to remove the interference substance.

13. The method for measuring a physiologically active substance of biological origin according to any one of claims 1 to 12, which is characterized that beads adsorbed with a predetermined protein contained in the hemocyte extract of limulus on the surface are dispersed in the AL reagent.

14. The method for measuring a physiologically active substance of biological origin according to any one of claims 1 to 13, which is characterized that the physiologically active substance of biological origin is endotoxin or β glucan.

15. A microparticle capable of adsorbing a physiologically active substance on the surface, which is used in the method for measuring a physiologically active substance of biological origin according to any one of claims 1 to 14.

16. An extraction liquid for extracting a physiologically active substance from a medical device in the method for measuring a physiologically active substance of biological origin according to claim 2, which is characterized that the microparticles capable of adsorbing the physiologically active substance on the surface are previously dispersed in the extraction liquid.
